(19) Europäisches Patentamt
European Patent Office
Office européen des brevets

(11) Veröffentlichungsnummer: **0 399 411 B1**

# (12) EUROPÄISCHE PATENTSCHRIFT

(45) Veröffentlichungstag der Patentschrift: **13.07.94**

(51) Int. Cl.$^{5}$: **C09B 62/507**, C09B 62/006, C07C 317/36, D06P 1/38

(21) Anmeldenummer: **90109527.3**

(22) Anmeldetag: **19.05.90**

## (54) Wasserlösliche Azoverbindungen, Verfahren zu ihrer Herstellung und ihre Verwendung als Farbstoffe.

(30) Priorität: **23.05.89 DE 3916712**

(43) Veröffentlichungstag der Anmeldung:
**28.11.90 Patentblatt 90/48**

(45) Bekanntmachung des Hinweises auf die Patenterteilung:
**13.07.94 Patentblatt 94/28**

(84) Benannte Vertragsstaaten:
**BE CH DE ES FR GB IT LI**

(56) Entgegenhaltungen:
**EP-A- 0 219 080**
**FR-A- 2 139 127**

**CHEMICAL ABSTRACTS, Band 104, Nr. 8, Februar 1986, Seiten 64-65,Zusammenfassung Nr. 52061f, Columbus, Ohio, US;**

**& JP-A-60 163 972 (SUMITOMO CHEMICAL CO., LTD) 26-08-1985**

(73) Patentinhaber: **HOECHST AKTIENGESELLSCHAFT**

**D-65926 Frankfurt(DE)**

(72) Erfinder: **Springer, Hartmut, Dr.**
**Am Erdbeerstein 27**
**D-6240 Königstein/Taunus(DE)**
Erfinder: **Reiher, Uwe, Dr.**
**Koblenzer Strasse 52**
**D-6000 Frankfurt am Main(DE)**

EP 0 399 411 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung liegt auf dem Gebiet der faserreaktiven Farbstoffe.

Faserreaktive Farbstoffe sind beispielsweise aus der japanischen Patentmeldungs-Veröffentlichung Sho-85-163 972 (Chemical Abstracts 104, 52061f (1986) sowie aus EP-A-0 219 080 und FR-A-2 139 927 bekannt.

Es wurden neue wasserlösliche Azoverbindungen entsprechend der allgemeinen Formel (1)

$$D - N = N - K \quad (1)$$

gefunden, die wertvolle faserreaktive Farbstoffeigenschaften besitzen.

In dieser Formel (1) bedeuten:

D ist ein Rest der allgemeinen Formel (2)

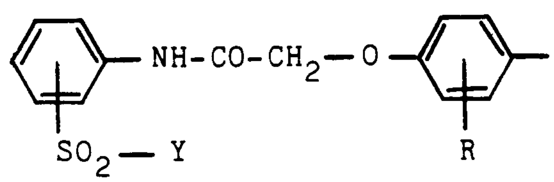

(2)

in welcher

Y die Vinylgruppe bedeutet oder eine Gruppe der allgemeinen Formel (3)

$$- CH_2 - CH_2 - X \quad (3)$$

in welcher

X ein Substituent ist, der durch ein Alkali unter Bildung der Vinylgruppe eliminierbar ist,

wobei die Gruppe $-SO_2-Y$ bevorzugt in meta- oder para-Stellung zur Amidocarbonylgruppe an den Benzolkern gebunden ist, und

R ein Wasserstoffatom, eine Nitrogruppe, eine Alkylgruppe von 1 bis 4 C-Atomen, wie Ethyl- und insbesondere Methylgruppe, eine Alkoxygruppe von 1 bis 4 C-Atomen, wie Ethoxy- und insbesondere Methoxygruppe, eine Carboxygruppe, eine Hydroxygruppe oder ein Halogenatom, wie Chlor- und Bromatom, bevorzugt jedoch ein Wasserstoffatom ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure, wie der Benzoesäure, oder einer aromatischen Sulfonsäure, wie der Benzol- oder Toluolsulfonsäure, oder einer N-substituierten Carbaminsäure, wie der N-Phenylureido-Rest, oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine oder mehrere faserreaktive Gruppen enthalten kann, wie bspw. eine Gruppe $-SO_2-Y$ mit Y der obigen Bedeutung oder eine 4-Fluor- oder 4-Chlor-6-amino-s-triazin-2-ylamino-Gruppe,deren 6-ständige Aminogruppe durch Alkyl von 1 bis 4 C-Atomen und/oder Phenyl mono- oder disubstituiert sein kann, wobei der Phenylrest durch Substituenten aus der Gruppe Sulfo, Carboxy, Methoxy, Ethoxy, Methyl, Chlor, Brom und $-SO_2-Y$ mit Y der obigen Bedeutung substituiert sein kann.

Alkalisch eliminierbare Substituenten X sind beispielsweise Halogenatome, wie das Bromatom und Chloratom, Estergruppen organischer Carbon- und Sulfonsäuren, wie ein Alkanoyloxyrest von 2 bis 5 C-Atomen, beispielsweise die Acetyloxygruppe, oder ein Sulfobenzoyloxy-, Benzoyloxy-, Phenylsulfonyloxy- oder Toluylsulfonyloxy-Rest, desweiteren beispielsweise die Phosphato-, Sulfato- und Thiosulfatogruppen, ebenso Dialkylaminogruppen mit Alkylgruppen von jeweils 1 bis 4 C-Atomen, wie die Dimethylamino- und Diethylaminogruppe.

Bevorzugt ist Y die Vinylgruppe und insbesondere die $\beta$-Sulfatoethyl-Gruppe.

Sulfogruppen sind Gruppen entsprechend der allgemeinen Formel $-SO_3M$ , Carboxygruppen sind Gruppen entsprechend der allgemeinen Formel -COOM , Sulfatogruppen sind Gruppen entsprechend der allgemeinen Formel $-OSO_3M$ , Thiosulfatogruppen sind Gruppen entsprechend der allgemeinen Formel $-S-SO_3M$ und Phosphatogruppen sind Gruppen entsprechend der allgemeinen Formel $-OPO_3M_2$ , in welchen

M ein Wasserstoffatom oder ein salzbildendes Metallatom, wie insbesondere ein Alkalimetallatom, wie beispielsweise Natrium, Kalium oder Lithium, ist.

Von den erfindungsgemäßen Verbindungen der allgemeinen Formel (1) können beispielsweise solche Verbindungen hervorgehoben werden, in welchen K einen Rest der nachstehenden Formel (4a), (4b), (4c), (4d), (4e), (4f), (4g), (4h), (4i), (4k), (4m), (4n), (4p), (4q), (4r), (4s), (4t) (4v) und (4w) bedeutet:

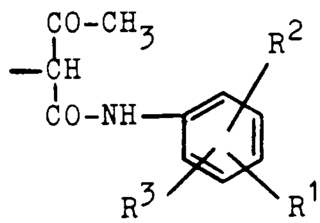

(4a)

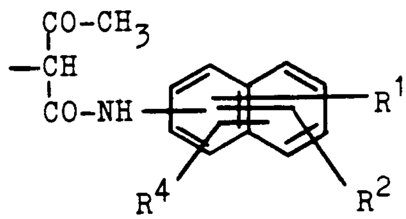

(4b)

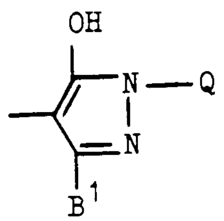

(4c)

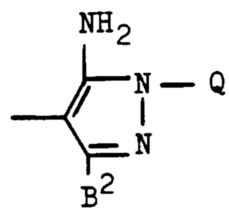

(4d)

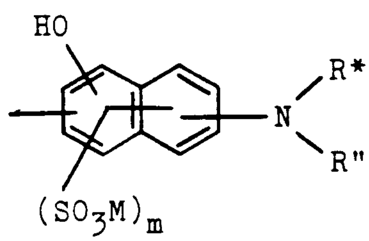

(4e)

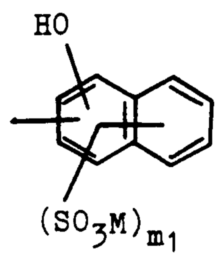

(4f)

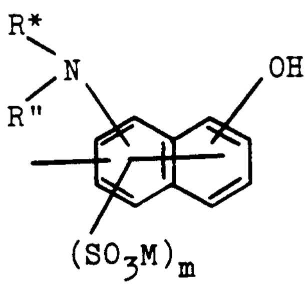

(4g)

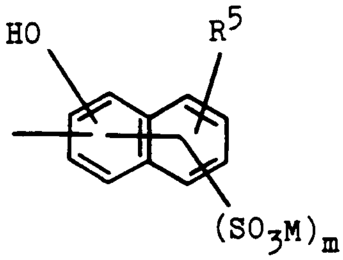

(4h)

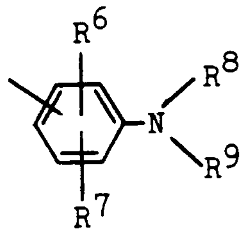

(4i)

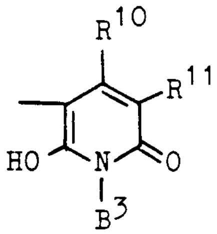

(4k)

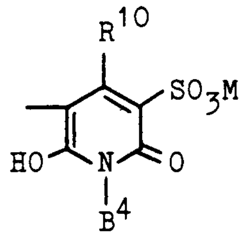

(4m)

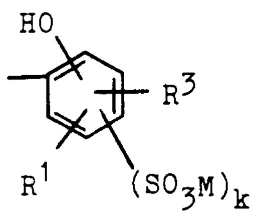

(4n)

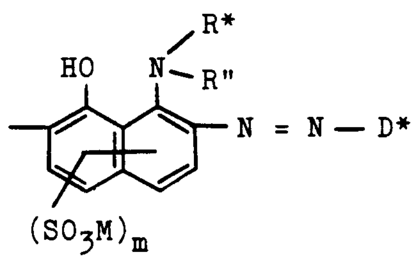

(4p)

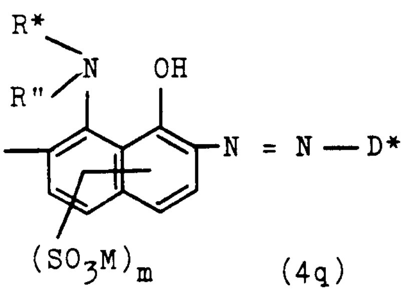

(4q)

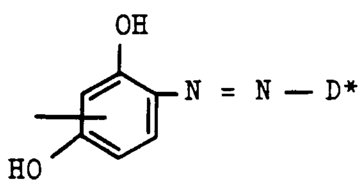

(4r)

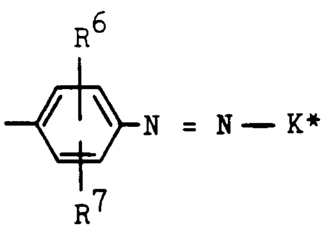

(4s)

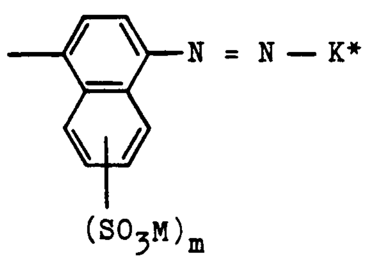

(4t)

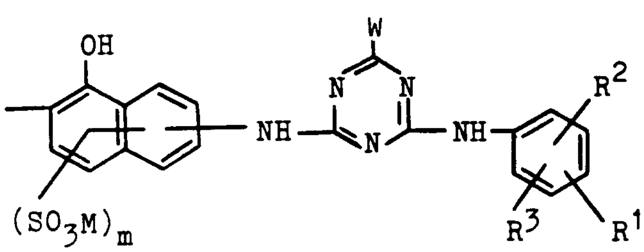

(4v)

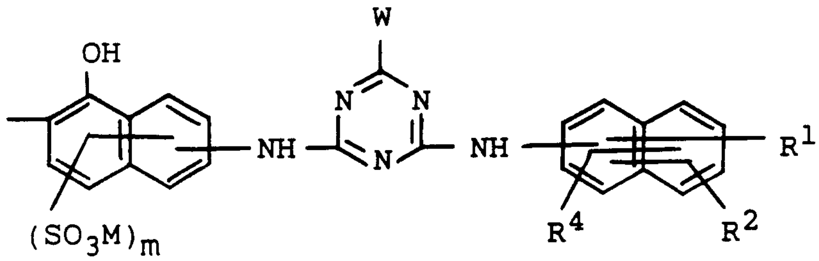

(4w)

In diesen Formeln bedeuten:

- $R^1$ ist Wasserstoff, Carboxy, Sulfo oder eine Gruppe der allgemeinen Formel $-SO_2-Y$ mit Y der obigen Bedeutung;
- $R^2$ ist Wasserstoff, Alkyl von 1 bis 4 C-Atomen, wie Methyl und Ethyl, Alkoxy von 1 bis 4 C-Atomen, wie Methoxy und Ethoxy, Chlor, Brom, Carboxy, Sulfo oder Nitro;
- $R^3$ ist Wasserstoff, Alkyl von 1 bis 4 C-Atomen, wie Methyl und Ethyl, Alkoxy von 1 bis 4 C-Atomen, wie Methoxy und Ethoxy, Chlor oder Brom;
- $R^4$ ist Wasserstoff, Sulfo oder Carboxy, bevorzugt Wasserstoff, falls $R^1$ eine Gruppe $-SO_2-Y$ ist;
- $B^1$ ist Alkyl von 1 bis 4 C-Atomen, wie Methyl, Carboxy, Carbalkoxy 2 bis 5 C-Atomen, wie Carbomethoxy und Carbethoxy, Carbamoyl, Phenyl oder durch Sulfo, Carboxy, Methyl, Ethyl, Methoxy, Ethoxy und/oder Chlor substituiertes Phenyl;
- $B^2$ ist Alkyl von 1 bis 4 C-Atomen, wie Methyl, Carboxy, Carbalkoxy von 2 bis 5 C-Atomen, wie Carbomethoxy und Carbethoxy, Carbamoyl, Phenyl oder durch 1 oder 2 Substituenten aus der Gruppe Alkyl von 1 bis 4 C-Atomen, Alkoxy von 1 bis 4 C-Atomen, Chlor, Brom und Sulfo substituiertes Phenyl;
- Q ist Phenyl, das substituiert sein kann, wie beispielsweise durch 1, 2 oder 3, bevorzugt 1 oder 2, Substituenten aus der Gruppe Chlor, Brom, Methyl, Ethyl, Methoxy, Ethoxy, Carboxy, Sulfo und Alkanoylamino von 2 bis 5 C-Atomen, wie Acetylamino, und/oder durch eine Gruppe der allgemeinen Formel $-SO_2-Y$ mit Y der obengenannten Bedeutung, oder ist Naphthyl, das durch 1, 2 oder 3 Sulfo und gegebenenfalls durch 1 Alkyl von 1 bis 4 C-Atomen, 1 Alkoxy von 1 bis 4 C-Atomen, 1 Chlor oder 1 Alkanoylamino von 2 bis 5 C-Atomen und/oder durch eine Gruppe der allgemeinen Formel $-SO_2-Y$ mit Y der obengenannten Bedeutung substituiert sein kann;
- $R^*$ ist Wasserstoff oder Alkyl von 1 bis 4 C-Atomen, das durch Phenyl oder durch Sulfo und/oder $-SO_2-Y$ mit Y der obigen Bedeutung substituiertes Phenyl substituiert sein kann;
- $R''$ ist Wasserstoff oder Alkyl von 1 bis 4 C-Atomen, das durch Phenyl, Sulfophenyl oder eine Gruppe der Formel $-SO_2-Y$ mit Y der obigen Bedeutung substituiert sein kann, oder ist Phenyl oder durch 1 oder 2 Substituenten aus der Gruppe Alkyl von 1 bis 4 C-Atomen, Alkoxy von 1 bis 4 C-Atomen, Chlor, Brom, Sulfo und $-SO_2-Y$ mit Y der obigen Bedeutung substituiertes Phenyl;
- $R^5$ ist Phenylureido, dessen Phenylrest durch eine Gruppe der Formel $-SO_2-Y$ mit Y der obigen Bedeutung substituiert sein kann, oder ist Alkanoylamino von 2 bis 5 C-Atomen, wie Acetylamino oder Propionylamino, das im Alkylrest durch eine Gruppe der Formel $-SO_2-Y$ mit Y der obigen Bedeutung substituiert sein kann, oder ist Alkenoylamino von 3 bis 5 C-Atomen, wie Acryloylamino, oder ist Benzoylamino, das durch Substituenten aus der Gruppe Chlor, Methyl, Methoxy, Nitro, Sulfo, Carboxy und $-SO_2-Y$ mit Y der obigen Bedeutung substituiert sein kann, und ist bevorzugt Acetylamino oder Benzoylamino;
- $R^6$ ist Wasserstoff, Alkyl von 1 bis 4 C-Atomen, Alkoxy von 1 bis 4 C-Atomen, Sulfo, Carboxy, Carbalkoxy von 2 bis 5 C-Atomen, wie Carbomethoxy und Carbethoxy, Halogen, wie Brom oder Chlor, oder Alkoxy von 1 bis 4 C-Atomen, das durch Hydroxy, Acetyloxy, Carboxy, Carbamoyl, Cyano oder Halogen, wie Chlor, substituiert ist;
- $R^7$ ist Wasserstoff, Alkyl von 1 bis 4 C-Atomen, Alkoxy von 1 bis 4 C-Atomen, Halogen, wie Brom oder Chlor, Cyano, Trifluormethyl, Alkoxy von 1 bis 4 C-Atomen, das durch Hydroxy, Acetyloxy, Carboxy, Carbamoyl oder Cyano oder Halogen, wie Chlor, oder durch eine Gruppe der Formel $-SO_2-Y$ mit Y der obigen Bedeutung substituiert ist, oder ist Alkanoylamino von 2 bis 5 C-Atomen, das durch Chlor, Brom, Alkoxy von 1 bis 4 C-Atomen, Phenoxy, Phenyl, Hydroxy, Carboxy oder Sulfo oder eine Gruppe der Formel

-$SO_2$-Y mit Y der obigen Bedeutung substituiert sein kann, oder ist Alkenoylamino von 3 bis 5 C-Atomen, das durch Chlor, Brom, Carboxy oder Sulfo substituiert sein kann, oder ist Benzoylamino, das im Benzolkern substituiert sein kann, beispielsweise durch Substituenten aus der Gruppe Chlor, Methyl, Sulfo und eine Gruppe der Formel -$SO_2$-Y mit Y der obigen Bedeutung, oder ist Alkylsulfonyl von 1 bis 4 C-Atomen oder Phenylsulfonyl, das im Benzolkern substituiert sein kann, beispielsweise durch Substituenten aus der Gruppe Chlor, Methyl, Sulfo und eine Gruppe der Formel -$SO_2$-Y mit Y der obigen Bedeutung, oder ist Alkylsulfonylamino von 1 bis 4 C-Atomen, das durch Hydroxy, Sulfato, Chlor, Brom, Alkoxy von 1 bis 4 C-Atomen oder eine Gruppe der Formel -$SO_2$-Y mit Y der obigen Bedeutung substituiert sein kann, oder ist Phenylsulfonylamino, das im Benzolkern substituiert sein kann, beispielsweise durch Substituenten aus der Gruppe Chlor, Methyl, Sulfo und eine Gruppe der Formel -$SO_2$-Y mit Y der obigen Bedeutung, oder ist Carbamoyl, das am Stickstoffatom durch 1 oder 2 Substituenten mono- oder disubstituiert sein kann, wobei die Substituenten der Gruppe Alkyl von 1 bis 4 C-Atomen, durch beispielsweise Hydroxy, Sulfo, Carboxy, Sulfato oder Phenyl oder eine Gruppe der Formel -$SO_2$-Y mit Y der obigen Bedeutung substituiertes Alkyl von 1 bis 4 C-Atomen, Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel -$SO_2$-Y mit Y der obigen Bedeutung, substituiertes Phenyl angehören, oder ist Sulfamoyl, das am Stickstoffatom durch 1 oder 2 Substituenten mono- oder disubstituiert sein kann, wobei die Substituenten der Gruppe Alkyl von 1 bis 4 C-Atomen, durch Substituenten, beispielsweise aus der Gruppe Hydroxy, Sulfo, Carboxy, Sulfato oder Phenyl oder eine Gruppe der Formel -$SO_2$-Y mit Y der obigen Bedeutung, substituiertes Alkyl von 1 bis 4 C-Atomen, Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel -$SO_2$-Y mit Y der obigen Bedeutung substituiertes Phenyl angehören, oder ist Ureido oder Ureido, das am endständigen Stickstoffatom durch 1 oder 2 Substituenten mono- oder disubstituiert sein kann, wobei die Substituenten der Gruppe Alkyl von 1 bis 4 C-Atomen, durch beispielsweise Hydroxy, Sulfo, Carboxy, Sulfato, Phenyl oder eine Gruppe der Formel -$SO_2$-Y mit Y der obigen Bedeutungen substituiertes Alkyl von 1 bis 4 C-Atomen, Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel -$SO_2$-Y mit Y der obigen Bedeutung, substituiertes Phenyl angehören;

$R^8$ ist Wasserstoff oder Alkyl von 1 bis 4 C-Atomen, das durch beispielsweise Hydroxy, Sulfo, Carboxy, Sulfato, eine Gruppe -$SO_2$-Y mit Y der obigen Bedeutung, Phenyl oder Sulfophenyl substituiert sein kann, oder ist Alkenyl von 2 bis 4 C-Atomen, das durch Carboxy, Sulfo, Chlor oder Brom substituiert sein kann, oder ist Cycloalkyl von 5 bis 8 C-Atomen;

$R^9$ ist Wasserstoff oder Alkyl von 1 bis 4 C-Atomen, das substituiert sein kann, beispielsweise durch Hydroxy, Sulfo, Carboxy, Sulfato, Phenyl oder -$SO_2$-Y mit Y obiger Bedeutung, oder ist Alkenyl von 2 bis 5 C-Atomen, das durch Carboxy, Sulfo oder -$SO_2$-Y mit Y obiger Bedeutung oder durch Chlor oder Brom substituiert sein kann, oder

$R^9$ ist Cycloalkyl von 5 bis 8 C-Atomen oder Phenyl, das substituiert sein kann, beispielsweise durch Substituenten aus der Gruppe Chlor, Sulfo, Methyl, Methoxy, Carboxy und -$SO_2$-Y mit Y obiger Bedeutung, oder

$R^8$ und $R^9$ stellen zusammen mit dem Stickstoffatom und gegebenenfalls einem weiteren Heteroatom oder einer Heterogruppe, wie N, O, S und NH, einen 5- bis 8-gliedrigen, bevorzugt gesättigten, heterocyclischen Rest dar, wie beispielsweise den N-Piperidino-, N-Morpholino- oder N-Piperazinorest;

$R^{10}$ ist Wasserstoff oder Alkyl von 1 bis 4 C-Atomen oder durch Alkoxy von 1 bis 4 C-Atomen oder Cyan substituiertes Alkyl von 1 bis 4 C-Atomen;

$R^{11}$ ist Wasserstoff, Sulfo, Sulfoalkyl mit einem Alkylenrest von 1 bis 4 C-Atomen, wie Sulfomethyl, Cyano oder Carbamoyl;

$B^3$ ist Wasserstoff oder Alkyl von 1 bis 6 C-Atomen, bevorzugt von 1 bis 4 C-Atomen, das durch Phenyl, Sulfo, Sulfophenyl oder -$SO_2$-Y mit Y obiger Bedeutung substituiert sein kann;

$B^4$ ist Wasserstoff, Alkyl von 1 bis 4 C-Atomen oder durch Alkoxy von 1 bis 4 C-Atomen, wie Methoxy, Sulfo, Carboxy, Sulfato, Acetylamino, Benzoylamino oder Cyano oder durch eine Gruppe der Formel -$SO_2$-Y mit Y obiger Bedeutung substituiertes Alkyl von 1 bis 4 C-Atomen oder ist Alkenyl von 2 bis 4 C-Atomen, Cyclohexyl, Phenyl oder durch Substituenten aus der Gruppe Carboxy, Sulfo, Benzoylamino, Acetylamino, $-SO_2-Y$ mit Y obiger Bedeutung und Chlor substituiertes Phenyl;

k ist die Zahl Null oder 1 (wobei im Falle k = Null diese Gruppe für ein Wasserstoffatom steht);

m ist die Zahl 1 oder 2;

$m_1$ ist die Zahl 1, 2 oder 3;

D* ist eine Gruppe der allgemeinen Formel (2) oder ist Phenyl, das durch 1, 2 oder 3, bevorzugt 1 oder 2, Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen, bevorzugt hiervon Methyl, Methoxy, Ethoxy, Chlor, Sulfo, Carboxy und Hydroxy, und/oder durch eine Gruppe der Formel $-SO_2-Y$ mit Y der obengenannten Bedeutung substituiert sein kann, wobei bevorzugt einer dieser Substituenten eine Sulfo- oder Carboxygruppe ist und die Gruppe $-SO_2-Y$ bevorzugt in meta- oder para-Stellung zur Azogruppe steht, oder D* ist Naphthyl, das durch 1, 2 oder 3 Sulfogruppen oder durch 1 oder 2 Sulfogruppen und 1 oder 2 Gruppen der allgemeinen Formel $-SO_2-Y$ mit Y der obengenannten Bedeutung oder nur durch eine solche Gruppe $-SO_2-Y$ substituiert ist,

wobei D und D* zueinander gleiche oder voneinander verschiedene Bedeutungen besitzen können;

K* ist ein Rest aus einer der oben genannten und definierten allgemeinen Formeln (4a) bis (4m), wobei K und K* zueinander gleiche oder voneinander verschiedene Bedeutungen besitzen können;

W ist Sulfo, Alkylsulfonyl von 1 bis 4 C-Atomen, Phenylsulfonyl oder Brom oder bevorzugt Fluor oder Chlor;

M hat eine der oben genannten Bedeutungen.

Die einzelnen Formelglieder, auch die gegebenenfalls in ein und derselben Formel zweifach auftretenden Formelglieder, können zueinander gleiche oder voneinander verschiedene Bedeutungen besitzen.

Die in den obigen Formeln (4e), (4f), (4g), (4h), (4i) und (4n) befindlichen freien Bindungen, welche zur Azogruppe führen, bzw. die Azogruppe in Formel (4p) und (4q) befinden sich in ortho-Stellung zur Hydroxy- bzw. Aminogruppe gebunden. Bevorzugt steht diese Hydroxygruppe in $\alpha$-Stellung an den Naphthalinrest gebunden.

Alkylgruppen von 1 bis 4 C-Atomen sind bevorzugt die Ethyl- und insbesondere die Methylgruppe; Alkoxygruppen von 1 bis 4 C-Atomen sind bevorzugt die Ethoxy- und insbesondere die Methoxygruppe; Alkanoylaminogruppen von 2 bis 5 C-Atomen sind bevorzugt die Propionylaminogruppe und insbesondere die Acetylaminogruppe und Carbalkoxygruppen von 2 bis 5 C-Atomen, bevorzugt die Carbomethoxy- und Carbethoxygruppe.

Von den erfindungsgemäßen Verbindungen der allgemeinen Formel (1) sind insbesondere diejenigen bevorzugt, in welchen K einen Rest der allgemeinen Formel (4c), (4f), (4h), (4p) oder (4q) bedeutet, in welchen wiederum die einzelnen Formelglieder die folgenden bevorzugten Bedeutungen besitzen:

$B^1$ ist Carboxy oder Methyl;

Q ist Phenyl, das durch 1 oder 2 Substituenten substituiert sein kann, die aus der folgenden Menge an Substituenten ausgewählt sind: 2 Methyl, 2 Methoxy, 1 Chlor oder Brom, 2 Sulfo, 1 Carboxy und 1 Vinylsulfonyl oder $\beta$-Sulfatoethylsulfonyl;

$R^5$ ist Acetylamino, Propionylamino oder Benzoylamino, das durch 1 oder 2 Substituenten aus der Gruppe Chlor, Methyl, Methoxy, Nitro, Sulfo und $\beta$-Sulfatoethylsulfonyl substituiert sein kann;

R* und R″ sind beide Wasserstoff;

m ist in Formel (4p) und (4q) die Zahl 2, und die eine Gruppe $-SO_3M$ steht in meta-Stellung zur Hydroxygruppe und die andere Gruppe $-SO_3M$ in meta- oder para-Stellung zur Aminogruppe.

Insbesondere bevorzugt sind solche Verbindungen der allgemeinen Formel (1), in welchen K den durch 1, 2 oder 3 Sulfogruppen substituierten 1-Hydroxy-naphth-2-yl-Rest darstellt oder einen Rest der allgemeinen Formel (4c) bedeutet, in welcher $B^1$ eine Carboxy- oder Methylgruppe ist und Q für einen Phenylrest steht, der durch 1 oder 2 Substituenten substituiert ist, die aus der Gruppe von 2 Methylgruppen, 2 Ethoxygruppen, 2 Methoxygruppen, 2 Sulfogruppen, 1 Carboxygruppe oder 1 Chloratom ausgewählt sind, wobei einer der Substituenten zwingend eine Carboxy- oder Sulfogruppe ist, oder der Phenylrest durch eine Vinylsulfonyl- oder $\beta$-Sulfatoethylsulfonyl-Gruppe substituiert ist und zusätzlich durch 1 oder 2 Substituenten substituiert sein kann, die aus der Gruppe von 1 Methyl, 2 Methoxy, 1 Chlor und 1 Sulfo ausgewählt sind.

In der Komponente K der allgemeinen Formel (4v) ist $R^1$ bevorzugt eine Gruppe $-SO_2$-Y mit Y der obengenannten, insbesondere bevorzugten Bedeutung, $R^2$ ein Wasserstoffatom, eine Alkylgruppe von 1 bis 4 C-Atomen, eine Alkoxygruppe von 1 bis 4 C-Atomen, ein Chlor- oder Bromatom oder eine Carboxy-, Sulfo- oder Nitrogruppe und $R^3$ ein Wasserstoffatom.

Die vorliegende Erfindung betrifft weiterhin Verfahren zur Herstellung der erfindungsgemäßen Azoverbindungen der allgemeinen Formel (1), beispielsweise durch Kupplungsreaktion der Diazoniumverbindung einer Aminoverbindung der allgemeinen Formel (5)

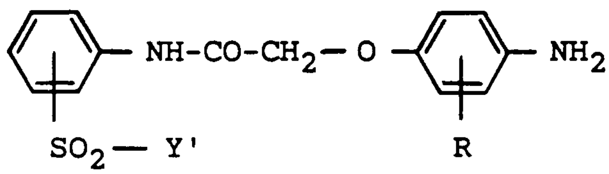

(5)

in welcher Y′ eine der Bedeutungen von Y hat oder die β-Hydroxyethyl-Gruppe ist und R die obengenannte Bedeutung besitzt, mit einer Kupplungskomponente der allgemeinen Formel H-K mit K der obengenannten Bedeutung; sofern K, wie oben angegeben, eine bivalente (Kupplungskomponente ist, kann eine Disazoverbindung, sofern diese erwünscht ist, durch Umsetzung dieser bivalenten Kupplungskomponente mit der zweifach äquimolaren Menge der Diazokomponente hergestellt werden. Im Falle der Verwendung einer Verbindung (5) mit Y′ gleich einer β-Hydroxyethyl-Gruppe wird diese β-Hydroxyethylgruppe in der gebildeten Azoverbindung in eine Gruppe Y der erfindungsgemäßen Azoverbindung (1), wie später noch angegeben, übergeführt.

Die Diazotierungs- und Kupplungsreaktionen erfolgen in üblicher und altbekannter Weise, so die Diazotierung des Amins (5) in der Regel bei einer Temperatur zwischen -5 °C und +15 °C und einem pH-Wert unterhalb von 2 mittels einer starken Säure und Alkalinitrit in bevorzugt wäßrigem Medium und die Kupplungsreaktion in der Regel bei einem pH-Wert zwischen 1,5 und 4,5 im Falle einer aminogruppenhaltigen Kupplungskomponente und bei einem pH-Wert zwischen 3 und 7,5 im Falle einer hydroxygruppenhaltigen Kupplungskomponente und bei einer Temperatur zwischen 0 und 30 °C in bevorzugt wäßrigem Medium.

Ist die Kupplungskomponente eine bivalente, doppelankuppelbare Verbindung, enthält sie beispielsweise eine kupplungsfähige Aminogruppe und gleichzeitig eine kupplungsfähige Hydroxygruppe, so kann zur Herstellung einer Disazoverbindung die Kupplung zunächst mit dem ersten Mol der Diazoniumverbindung des Amins im sauren pH-Bereich zur Monoazoverbindung erfolgen und die zweite Kupplungsreaktion mit dem zweiten Mol der Diazoniumverbindung des Amins anschließend im schwach sauren bis schwach alkalischen Bereich. Diese Verfahrensweise gilt beispielsweise für die Verbindungen entsprechend den allgemeinen Formeln (4p) und (4q), so durch Kupplung der Aminonaphtholsulfonsäure zunächst mit dem ersten Mol der Diazoniumverbindung des Amins der allgemeinen Formel (5) oder eines anderen aromatischen eins entsprechend der allgemeinen Formel $D^*-NH_2$ mit D* der obengenannten anderen Bedeutung als D im sauren Medium und sodann durch Kupplung der gebildeten Monoazoverbindung mit dem zweiten Mol einer Diazoniumverbindung eines eins $D^*-NH_2$ mit D* der obengenannten Bedeutung im schwach sauren, neutralen oder schwach alkalischen Bereich, wobei D* zwingend eine der für D angegebenen Bedeutungen besitzt, sofern die erste Kupplungsreaktion nicht mit einer Diazoniumverbindung eines eins (5) durchgeführt wurde, so insbesondere zunächst bei einem pH-Wert von etwa 1 bis 2,5 und anschließend bei einem pH-Wert zwischen 4 und 6,5, wobei, sofern die Diazoniumverbindung der Aminoverbindung (5) in beiden Kupplungsreaktionen identisch ist, die erste und zweite Kupplungsreaktion in ein und demselben Ansatz, zunächst im angegebenen sauren Bereich und sodann im schwach sauren bis schwach alkalischen Bereich, durchgeführt werden kann. Zur Herstellung einer Disazoverbindung entsprechend der allgemeinen Formel (4r) erfolgt die Umsetzung der Kupplungskomponente Resorcin mit der bzw. den Diazoniumverbindung(en) vorteilhaft zunächst bei einem pH-Wert zwischen 0,8 und 2 und sodann bei einem pH-Wert zwischen 6 und 7,5.

Disazoverbindungen entsprechend der allgemeinen Formel (1), deren Rest K dem Rest einer Azoverbindung entspricht, welcher aus einer kupplungsfähigen Diazokomponente und einer Kupplungskomponente zusammengesetzt ist, wie beispielsweise ein Rest entsprechend der allgemeinen Formel (4s) oder (4t), lassen sich auch erfindungsgemäß in der Weise herstellen, daß man zunächst die Diazoniumverbindung eines Amins (5) mit der aminogruppenhaltigen und somit diazotierbaren Kupplungskomponente, wie beispielsweise in den Formeln (4s) und (4t) die durch die Substituenten $R^6$ und $R^7$ substituierten Anilin- bzw. Sulfo-aminonaphthalin-Komponenten, kuppelt und in der so gebildeten Amino-Azoverbindung die

Aminogruppe diazotiert und mit einer Kupplungskomponente, wie beispielsweise der Kupplungskomponente H-K* , zur Disazoverbindung kuppelt.

Alle diese Umsetzungsmöglichkeiten zur Synthese von Disazoverbindungen sind analog den in der Literatur bekannten oder dem Fachmann geläufigen Methoden zur Synthese von Disazoverbindungen.

Kupplungskomponenten, die zur Herstellung der erfindungsgemäßen Farbstoffe verwendet werden können und beispielsweise den allgemeinen Formeln (4a) bis (4n) entsprechen, sind beispielsweise: 1,3-Diamino-benzol-5-sulfonsäure, Phenol, Kresol, Resorcin, 2-Ethoxy-phenol, 4-Methylphenol, 3-Sulfophenol, Salicylsäure, 3-Sulfo-1-naphthol, 4-Sulfo-1-naphthol, 5-Sulfo-1-naphthol, 3,6-Disulfo-8-naphthol, 4,6-Disulfo-8-naphthol,1-Naphthol-3,8-disulfonsäure, 1-Amino-8-naphthol-4-sulfonsäure, 1-Amino-8-naphthol-5-sulfonsäure, 1-Amino-8-naphthol-2,4-disulfonsäure, 2-Amino-5-naphthol-7-sulfonsäure, 2-Amino-5-naphthol-1,7-disulfonsäure, 1-Amino-5-naphthol-7-sulfonsäure, 2-Amino-8-naphthol-6-sulfonsäure, 2-Amino-8-naphthol-3,6-disulfonsäure, 2-Amino-8-naphthol-4,6-disulfonsäure, 1-Amino-8-naphthol-3,6- oder -4,6-disulfonsäure, 1-Acryloylamino-8-naphthol-3,6- oder -4,6-disulfonsäure, 1-Propionylamino-8-naphthol-3,6- oder -4,6-disulfonsäure, 1-Acetylamino-8-naphthol-4-sulfonsäure, 1-Acetylamino-8-naphthol-3,6- oder -4,6-disulfonsäure, 1-Benzoylamino-8-naphthol-3,6- oder -4,6-disulfonsäure, 2-Naphthol-5,7-disulfonsäure, 2-Naphthol-3,6-und -6,8-disulfonsäure, 1,8-Dihydroxynaphthalin-3,6-disulfonsäure, 1,8-Dihydroxynaphthalin-6-sulfonsäure, 1-Naphthol-3,6,8-trisulfonsäure,2-Acetylamino-5-naphthol-7-sulfonsäure, 2-Benzoylamino-8-naphthol-6-sulfonsäure, 2-(p′-Tosylamino)-5-naphthol-7-sulfonsäure, 2-Acetylamino-8-naphthol-3,6-disulfonsäure, 2-Acetylamino-5-naphthol-1,7-disulfonsäure, 3-Benzoylamino-8-naphthol-6-sulfonsäure, 2-Phenylsulfonylamino-5-naphthol-7-sulfonsäure, 2-(N-Methyl-N-acetyl)-amino-8-naphthol-6-sulfonsäure, N-Ethyl-N-benzylanilin-3-sulfonsäure, N,N-Bis-(β-hydroxyethyl)-anilin, N,N-Bis-(β-sulfatoethyl)-anilin, N,N-Bis-(β-hydroxyethyl)-2-methoxy-5-chlor-anilin, N-(β-Sulfatoethyl)-2,5-dimethoxy-anilin, N-(β-Sulfatoethyl)-2-chloranilin, Acetoacetyl-2-naphthylamid-5-sulfonsäure, N-Acetoacetylanilin-3- oder -4-sulfonsäure, N-Acetoacetyl-2-methoxy-5-sulfo-anilin, N-Acetoacetyl-4-methoxy-3-sulfoanilin, N-Acetoacetyl-2-methoxy-5-methyl-4-sulfo-anilin, N-Acetoacetyl-2,5-dimethoxy-4-sulfo-anilin, N-Acetoacetyl-2-methoxy-5-methyl-4-(β-sulfatoethylsulfonyl)-anilin, N-Acetoacetyl-2,5-dimethoxy-4-(β-sulfatoethylsulfonyl)-anilin, N-Acetoacetyl-2-methoxy-5-(β-sulfatoethylsulfonyl)-anilin, N-Acetoacetyl-4-(β-sulfatoethylsulfonyl)-anilin, N-Acetoacetyl-3-(β-sulfatoethylsulfonyl)-anilin, 1-(4′-β-Sulfatoethylsulfonyl-phenyl)-3-methylpyrazolon(5), 1-(4′-β-Sulfatoethylsulfonyl-phenyl)-3-carboxy-pyrazolon(5), 1-(4′-Sulfophenyl)-3-methyl-pyrazolon(5), 1-(4′-Sulfophenyl)-3-carboxy-pyrazolon(5), 1-(2′-Chlor-5′sulfo-phenyl)-3-methyl- oder -3-carboxy-pyrazolon(5), 1-(3′-Sulfophenyl)-3-carboxypyrazolon(5), 1-(2′-Methoxy-4′-sulfophenyl)-3-carboxy-pyrazolon(5), 1-(3′-Sulfophenyl)-3-methyl-5-amino-pyrazol, 1-(4′-Sulfo-phenyl)-3-methyl-5-amino-pyrazol, 1-(2′-Methoxy-5′-sulfo-phenyl)-3-methyl-5-aminopyrazol, 1-(2′-Methoxy-5′-methyl-4′-sulfophenyl)-3-methyl-5-aminopyrazol, 1-(2′-Chlor-5′-sulfo-phenyl)-3-methyl-5-aminopyrazol, 1-(3′-Amino-4′-sulfo-phenyl)-3-carbethoxy-pyrazolon(5), 1-(4′-β-Sulfatoethylsulfonyl-phenyl)-3-carbethoxy-pyrazolon(5), 1-(3′-Amino-6′-methyl-phenyl)-3-carboxy-pyrazolon-(5), 2-N-Methylamino-8-naphthol-6-sulfonsäure, 3-Carboxy-pyrazolon-(5),1-Phenyl-3-carboxy-pyrazolon(5), 1-(4′-Nitrophenyl)-3-carboxypyrazolon(5), 1-(3′-Acetylaminophenyl)-3-carboxy-pyrazolon(5), 1-(3′-Carboxyphenyl)-3-methyl-pyrazolon(5), 2-Hydroxy-3-carboxy-naphthalin, 2-Hydroxy-6-carboxy-naphthalin, 8-Hydroxy-chinolin-5-sulfonsäure, 1,4-Dimethyl-2-hydroxy-6-pyridon-5-sulfonsäure, N-Sulfomethyl-anilin, 3-Acetylamino-5-naphthol-7-sulfonsäure, 2-Methylamino-8-naphthol-6-sulfonsäure, 2,5-Disulfo-diphenylamin, 4-Sulfo-diphenylamin, 1-[4′-Chlor-6′-(4″-β-sulfatoethylsulfonyl-phenyl)-amino-1′,3′,5′-triazin-2′-yl]-amino-8-naphthol-3,6-disulfonsäure, 1-[4′-Chlor-6′-(4″-β-sulfatoethylsulfonyl-phenyl)-amino-1′,3′,5′-triazin-2′-yl]-amino-8-naphthol-4,6-disulfonsäure, 2-[4′-Chlor-6′-(4″-β-sulfatoethylsulfonyl-phenyl)-amino-1′,3′,5′-triazin-2′-yl]-amino-8-naphthol-6-sulfonsäure, 3-[4′-Chlor-6′-(4″-β-sulfatoethylsulfonyl-phenyl)-amino-1′,3′,5′-triazin-2′-yl]-amino-8-naphthol-3,6-disulfonsäure, 1-(4′-Chlor-6′-methoxy-1′,3′,5′-triazin-2′-yl)-amino-8-naphthol-3,6-disulfonsäure, 1-(4′-Chlor-6′-methoxy-1′,3′,5′-triazin-2′-yl)-amino-8-napthol-4,6-disulfonsäure, 2-(4′-Chlor-6′-methoxy-1′,3,′5′-triazin-2′-yl)-amino-8-naphthol-6-sulfonsäure, 3-(4′-Chlor-6′-methoxy-1′,3′,5′-triazin-2′-yl)-amino-8-naphthol-6-sulfonsäure, 1-[4′-Fluor-6′-(4″-β-Sulfatoethylsulfonyl-phenyl)-amino-1′,3′,5′-triazin-2′-yl]-amino-8-naphthol-3,6-disulfonsäure, 1-[4′-Fluor-6′-(4″-β-Sulfatoethylsulfonyl-phenyl)-amino-1′,3′,5′-triazin-2′-yl]-amino-8-naphthol-4,6-disulfonsäure, 2-[4′-Fluor-6′-(4″-β-Sulfatoethylsulfonyl-phenyl)-amino-1′,3′,5′-triazin-2′-yl]-amino-8-naphthol-6-sulfonsäure, 3-[4′-Fluor-6′-(4″-β-Sulfatoethylsulfonyl-phenyl)-amino-1′,3′,5′-triazin-2′-yl]-amino-8-naphthol-6-sulfonsäure, 1-(4′-β-Sulfatoethylsulfonyl-benzoyl)-amino-8-napthol-3,6-disulfonsäure oder -4,6-disulfonsäure, 2- oder 3-(4′-β-Sulfatoethylsulfonyl-benzoyl)-amino-8-naphthol-6-sulfonsäure, 1-{4′-Chlor-6′-[β-(4″-β″-sulfatoethylsulfonyl-phenyl)-ethyl]-1′,3′,5′-triazin-2′-yl}-amino-8-naphthol-3,6- oder -4,6-disulfonsäure, 1-{4′-Chlor-6′-[β-(3″-β″-sulfatoethylsulfonyl-phenyl)-ethyl]-1′,3′,5′-triazin-2′-yl}-amino-8-naphthol-3,6- oder -4,6-disulfonsäure, 1-{4′-Chlor-6′-[β-(4″-sulfo-phenyl)-ethyl]-1′,3′,5′-triazin-2′-yl}-amino-8-naphthol-3,6- oder -4,6-disulfonsäure, 1-{4′-Chlor-6′-[β-(2″,5″-disulfophenyl)-ethyl]-1′,3′,5′-triazin-2′-yl}-amino-8-naphthol-3,6 oder -4,6-disulfonsäure, 1-{4′-Fluor-6′-[β-(3″,5″-disulfophenyl)-ethyl]-1′,3′,5′-triazin-2′-yl}-amino-8-naphthol-3,6- oder -4, 6-disulfonsäure, 1-(β-Hydroxyethyl)-4-methyl-6-hydroxy-2-pyridon, 1-(β-Hydroxyethyl)-3-cyano-4-methyl-6-hydroxy-2-pyridon, 1-(β-Hydroxyethyl)-3-carbamoyl-4-methyl-6-hydroxy-2-pyridon, 1-(β-Hydroxyethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-cyano-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-Carboxymethyl-4-methyl-6-hydroxy-2-pyridon, 1-Carboxymethyl-3-cyano-4-methyl-6-hydroxy-2-pyridon, 1-Carboxymethyl-3-carbamoyl-4-methyl-6-hydroxy-2-pyridon, 1-Carboxymethyl-4-methyl-6-hydroxy-2-pyridon-3-sulfonsäure, 1-(β-Carboxyethyl)-4-methyl-6-hydroxy-2-pyridon, 1-(β Carboxyethyl)-3-cyano-4-methyl-6-hydroxy-2-pyridon, 1-(β-Carboxyethyl)-3-carbamoyl-4-methyl-6-hydroxy-2-pyridon, 1-(β-Carboxyethyl)-4-methyl-6-hydroxy-2-pyridon-2-sulfonsäure, 1-(β-Acetylaminoethyl)-4-methyl-6-hydroxy-2-pyridon, 1-(β-Acetylaminoethyl)-3-cyano-4-methyl-6-hydroxy-2-pyridon, 1-(β-Acetylaminoethyl)-3-carbamoyl-4-methyl-6-hydroxy-2-pyridon, 1-(β-Acetylaminoethyl)-4-methyl-6-hydroxy-2-pyridon-3-sulfonsäure, 1-(β-Acetylaminopropyl)-4-methyl-6-hydroxy-2-pyridon, 1-(β-Acetylaminopropyl)-3-cyano-4-methyl-6-hydroxy-2-pyridon, 1-(β-Acetylamino-propyl)-3-carbamoyl-4-methyl-6-hydroxy-2-pyridon, 1-(β-Acetylamino-propyl)-4-methyl-6-hydroxy-2-pyridon-3-sulfonsäure, 4-Hydroxy-chinolin-(2), 1-Amino-8-hydroxy-2-(phenylazo)-naphthalin-3,6-disulfonsäure, 1-Amino-8-hydroxy-2-(4′-sulfophenylazo)-naphthalin-3,6-disulfonsäure, 1-Amino-8-hydroxy-2-(2′,5′-disulfophenylazo)-naphthalin-3,6-disulfonsäure, 1-(β-Aminoethyl)-3-cyano-4-methyl-6-hydroxy-2-pyridon, 1-(γ-Aminopropyl)-3-sulfomethyl-4-methyl-6-hydroxy-2-pyridon, 1,3-Diaminobenzol, 1-Amino-3-(N,N-di-β-hydroxyethylamino)-benzol, 1-Amino-3-(N,N-di-β-sulfatoethylamino)-benzol, 1-Amino-(3-N,N-di-β-hydroxyethylamino)-4-methoxybenzol, 1-Amino-3-(N,N-di-β-sulfatoethylamino)-4-methoxy-benzol, 1-Amino-3-(sulfobenzylamino)-benzol, 1-Amino-3-(sulfobenzylamino)-4-chlorobenzol, 1-Amino-3-(N,N-di-sulfobenzylamino)-benzol, 1-Hydroxy-3- oder -4-methyl-benzol, 1-Hydroxybenzol-4-sulfosäure, 1-Hydroxynaphthalin, 2-Hydroxynaphthalin, 2-Hydroxynaphthalin-6- oder -7-sulfonsäure, 1-Hydroxynaphthalin-4,7-disulfonsäure, 1-Amino-3-methyl-benzol, 1-Amino-2-methoxy-5-methylbenzol, 1-Amino-2,5-dimethyl-benzol, 3-Aminophenylharnstoff, 1-Amino-3-acetylamino-benzol, 1-Amino-3-(hydroxyacetylamino)-benzol, 1,3-Diaminobenzol-4-sulfonsäure, 1-Amino-naphthalin-6- oder -8-sulfonsäure, 1-Amino-2-methoxy-naphthalin-6-sulfonsäure, 2-Amino-naphthalin-5,7-disulfonsäure, 1-Amino-8-hydroxy-naphthalin-6-sulfonsäure, 2-Hydroxy-3-aminonaphthalin-5,7-disulfonsäure, 1-Amino-8-hydroxy-naphthalin-2,4,6-trisulfonsäure, 1-Hydroxy-8-acetylamino-naphthalin-3-sulfonsäure, 1-Benzoylamino-8-hydroxy-naphthalin-3,6- oder -4,6-disulfonsäure, 2-Benzoylamino-5-hydroxy-naphthalin-7-sulfonsäure, 2-Methyl- und 2-Ethylamino-5-hydroxy-naphthalin-7-sulfonsäure, 2-(N-Acetyl-N-methylamino)-5-hydroxy-naphthalin-7-sulfonsäure, 2-Ethylamino-8-hydroxy-naphthalin-6-sulfonsäure, 2-Acetylamino-8-hydroxy-naphthalin-6-sulfonsäure, 1-(4′-Aminobenzoylamino)-8-hydroxynaphthalin-3,6- und -4,6-disulfonsäure, 1-(4′-Nitrobenzoylamino)-8-hydroxy-naphthalin-3,6- und -4,6-disulfonsäure, 1-(3′-Amino-benzoylamino)-6-hydroxy-naphthalin-3,6- und -4,6-disulfonsäure, 1-(3′-Nitrobenzoylamino)-8-hydroxy-naphthalin-3,6- und -4,6-disulfonsäure, 2-(4′-Amino-3′-sulfophenyl)-amino-5-hydroxy-naphthalin-7-sulfonsäure, 3-Methyl-5-pyrazolon, 1-Phenyl-3-methyl-5-pyrazolon, 1-(3′-Aminophenyl)-3-methyl-5-pyrazolon, 1-(2′,5′-Disulfophenyl)-3-methyl-5-pyrazolon, 1-(2′-Methyl-4′-sulfophenyl)-5-pyrazolon-3-carbonsäure, 1-(4′,8′-Disulfonaphthyl-2′-yl)-3-methyl-5-pyrazolon, 1-(5′,7′-Disulfonaphthyl-2-)-3-methyl-5-pyrazolon, 1-(2′,5′-Dichlor-4′-sulfophenyl)-3-methyl-5-pyrazolon, 3-Aminocarbonyl-4-methyl-6-hydroxy-2-pyridon, 1-Ethyl-3-cyano- oder -3-chlor-4-methyl-6-hydroxy-2-pyridon, 1-Ethyl-3-sulfomethyl-4-methyl-6-hydroxy-2-pyridon, 2,4,6-Triamino-3-cyano-pyridin, 2-(3′-Sulfophenyl)-amino-4,6-diamino-3-cyano-pyridin, 2-(2′-Hydroxyethylamino)-3-cyano-4-methyl-6-amino-pyridin, 2,6-Bis-(2′-hydroxyethylamino)-3-cyano-4-methyl-pyridin, 1-Ethyl-3-carbamoyl-4-methyl-6-hydroxy-2-pyridon, 1-Ethyl-3-sulfomethyl-4-methyl-5-carbamoyl-6-hydroxy-2-pyridon,N-Acetoacetylamino-benzol, 5-Acetylamino-2-sulfo-anilin.

Die erfindungsgemäß zur Synthese der erfindungsgemäßen Azoverbindungen (1) einsetzbaren Verbindungen entsprechend der allgemeinen Formel (5) sind bisher noch nicht bekannt. Die Erfindung betrifft somit auch diese Verbindungen, Verfahren zu deren Herstellung und ihre Verwendung zur Synthese von Farbstoffen, wie insbesondere zu den erfindungsgemäßen Azoverbindungen (1). Sie lassen sich analog bekannten Verfahrensweisen der Umsetzung von Säurechloriden mit Aminen herstellen, indem man zunächst eine Verbindung der allgemeinen Formel (6)

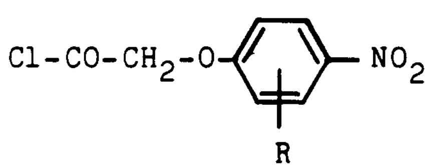

(6)

in welcher R die obengenannte Bedeutung hat, mit einer Aminoverbindung der Formel

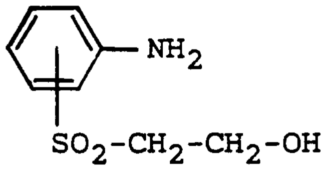

(7)

umsetzt. Die Umsetzung erfolgt in hierfür üblichen und geeigneten Löse- oder Verdünnungsmitteln und in Gegenwart eines säurebindenden Mittels, in der Regel bei einer Temperatur zwischen 50 und 80 °C. Geeignete Lösemittel sind beispielsweise Wasser oder ein organisches Löse- oder Verdünnungsmittel oder ein Gemisch von Wasser und einem Wasser mischbaren organischen Lösemittel. Organische Löse- oder Verdünnungsmittel sind beispielsweise Alkanole von 1 bis 6 C-Atomen, bevorzugt 1 bis 4 C-Atomen, wie beispielsweise Methanol, Dioxan, Toluol, die Xylole, Chlorbenzol, o-Dichlorbenzol, m-Dichlorbenzol, Dimethylformamid und N-Methyl-pyrrolidon. Säurebindende Mittel sind beispielsweise Kaliumcarbonat, Magnesiumoxid, Natriumcarbonat, Natriumhydroxid, Triethylamin und Triethanolamin. In wäßrigem Medium wird ein pH-Wert zwischen 6 und 12, bevorzugt zwischen 8 und 10, eingehalten.

Säurechloride der allgemeinen Formel (6) sind beispielsweise 4-Nitro-phenoxy-essigsäurechlorid, 2,4-Dinitro-phenoxy-essigsäurechlorid, 2-Chlor-4-nitro-phenoxy-essigsäurechlorid, 2-Brom-4-nitro-phenoxy-essigsäurechlorid, 2-Methyl-4-nitro-phenoxy-essigsäurechlorid und 2-Methoxy-4-nitro-phenoxy-essigsäurechlorid.

Aminoverbindungen der allgemeinen Formel (7) sind insbesondere 4-(β-Hydroxyethylsulfonyl)-anilin und 3-(β-Hydroxyethylsulfonyl)-anilin.

Die auf diese Weise erfindungsgemäß erhältlichen und ebenfalls neuen, erfindungsgemäßen Carbonamid-Verbindungen entsprechend der allgemeinen Formel (8)

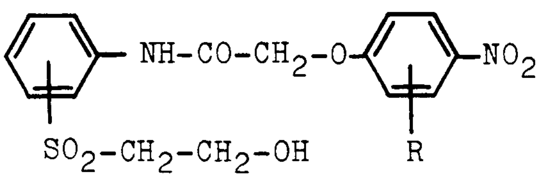

(8)

in welcher R die obengenannte Bedeutung hat, werden sodann analog bekannten Verfahrensweisen, nachdem sie beispielsweise aus dem Reaktionsansatz durch Kristallisation oder durch Abdestillieren des Lösemittels oder durch Ansäuern und Filtration isoliert wurden, zur Aminoverbindung entsprechend der allgemeinen Formel (5) reduziert, so durch katalytlsche Hydrierung mit Wasserstoff an Palladium, Platin oder Raney-Nickel bei einer Temperatur zwischen 50 und 110 °C und bei erhöhtem Druck oder durch Reduktion nach Béchamp mit Eisen in saurem Medium, beispielsweise mit Eisen in Ethanol/Eisessig. Die Reduktion kann in einem hierfür geeigneten Lösemittel, wie Wasser, Methanol oder Ethanol oder einer Mischung derselben, erfolgen.

Sowohl die Verbindungen entsprechend der allgemeinen Formel (1) als auch die der allgemeinen Formel (5), in welchen Y bzw. Y′ die β-Hydroxyethyl-Gruppe bedeuten, können in üblicher und bekannter Verfahrensweise in Verbindungen übergeführt werden, in welchen Y bzw. Y′ eine andere Bedeutung als die β-Hydroxyethyl-Gruppe besitzt, so beispielsweise in deren Esterderivate, wie beispielsweise von mehrwertigen anorganischen Säuren oder von aliphatischen und aromatischen Carbon- oder Sulfonsäuren, so beispielsweise in Verbindungen, in welchen Y bzw. Y′ für die β-Chlorethyl-, β-Sulfatoethyl-, β-phosphatoethyl-, β-Thiosulfatoethyl-, β-Acetyloxyethyl- oder β-Toluylsulfonyloxyethyl-Gruppe steht. Hierfür geeignete Veresterungs- und Acylierungsmittel sind beispielsweise die entsprechenden anorganischen oder organischen Säuren oder deren Anhydride oder Halogenide oder Amide, wie beispielsweise Schwefelsäure, Schwefeltrioxid enthaltende Schwefelsäure, Chlorsulfonsäure, Amidosulfonsäure, Phosphorsäure, Phosphoroxychlorid, Gemische aus Phosphorsäure und Phosphorpentoxid, Acetanhydrid, Toluolsulfochlorid und Thionylchlorid.

Diejenigen Verbindungen, in welchen Y bzw. Y′ für die Vinylgruppe steht, können aus deren analogen Esterderivaten mittels Alkali, so in wäßrigem Medium bei einem pH-Wert von 10 bis 12 und einer Temperatur zwischen 40 und 50 °C während 10 bis 20 Minuten, hergestellt werden. Die Synthese von beispielsweise β-(Dialkylamino)-ethylsulfonyl- und β-Thiosulfatoethylsulfonyl-Derivaten der Verbindungen (1)

und (5) erfolgt durch Umsetzung von deren Vinylsulfonyl-Verbindungen mit dem entsprechenden Dialkylamin oder mit einem Alkalisalz der Thioschwefelsäure, wie Natriumthiosulfat. Alle diese Verfahrensweisen der Überführung von einer Gruppe $-SO_2-Y$ bzw. $-SO_2-Y'$ in eine andere sind dem Fachmann auf diesem faserreaktiven Gebiet geläufig und zahlreich in der Literatur beschrieben.

Die erfindungsgemäßen Verbindungen der allgemeinen Formel (1) - im nachfolgenden als Verbindungen (1) bezeichnet - haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können die bei der Synthese der Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Die Abscheidung und Isolierung der Verbindungen (1) aus den wäßrigen Syntheselösungen kann nach allgemein bekannten Methoden für wasserlösliche Verbindungen erfolgen, so beispielsweise durch Ausfällen aus dem Reaktionsmedium mittels eines Elektrolyten, wie beispielsweise Natriumchlorid oder Kaliumchlorid, oder aber durch Eindampfen der Reaktionslösung selbst, beispielsweise durch Sprühtrocknung. Falls die letztgenannte Art der Isolierung gewählt wird, empfiehlt es sich vielfach, vor dem Eindampfen eventuell in den Lösungen vorhandene Sulfatmengen durch Fällung als Calciumsulfat und Abtrennung durch Filtration zu entfernen.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solche Färbe- und Fixierweisen sind in der Literatur zahlreich beschrieben (s. bspw. europäische Patentanmeldungs-Veröffentlichung Nr. 0 181 585 A2).

Die erfindungsgemäßen Färbungen besitzen, insbesondere auf Cellulosefasermaterialien, gute Lichtechtheiten sowohl im trockenen Zustand der Färbung als auch im nassen, beispielsweise mit einer Schweißlösung befeuchteten, Zustand sowie gute Naßechtheiten, wie beispielsweise gute Waschechtheiten bei 60 bis 95 °C, auch in Gegenwart von Perboraten, gute saure und alkalische Walk-, Überfärbe- und Schweißechtheiten, eine hohe Dampfbeständigkeit, gute Alkali-, Säure-, Wasser- und Seewasserechtheiten, des weiteren eine gute Plissierechtheit, Bügelechtheit und Reibechtheit. Ebenso besitzen sie eine gute Säurelagerbeständigkeit ("acid fading") beim Lagern von feuchten, noch Essigsäure enthaltendem, gefärbtem Material.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima ($\lambda_{max}$) im sichtbaren Bereich wurden anhand derer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

**Beispiel A**

Eine Lösung mit einem pH-Wert von 9 von 206 Teilen 4-(β-Hydroxyethylsulfonyl)-anilin in etwa 1500 Teilen Wasser wird langsam bei 5 bis 10 °C unter Einhaltung des pH-Wertes von 9 mittels etwa 3250 Teilen einer 20%igen wäßrigen Natriumcarbonatlösung unter gutem Rühren mit einer Lösung von 243 Teilen 4-Nitrophenoxyessigsäurechlorid in 500 Volumenteilen Toluol versetzt. Man rührt den Ansatz noch etwa 2 Stunden bei 5 bis 10 °C und anschließend noch 16 Stunden bei 20 °C nach, löst nicht umgesetztes Anilin durch Zugabe von 4000 Volumenteilen einer wäßrigen 5n-Salzsäure, saugt das nicht gelöste Produkt ab und kristallisiert es aus Methanol um.
Es besitzt die Formel

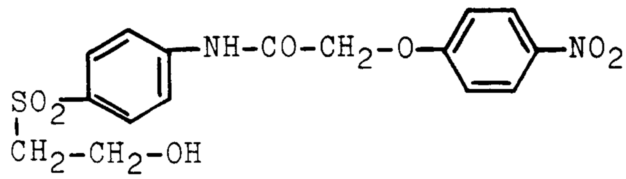

und hat folgende physikalische Daten:
Schmelzpunkt: 220 °C ;
Banden im IR-Spektrum:
3538 $cm^{-1}$ (OH); 3376 $cm^{-1}$ (NH); 2952 $cm^{-1}$ (CH); 1696 $cm^{-1}$ (CO).

**Beispiel B**

50 Teile der Nitroverbindung von Beispiel A werden in 300 Teilen Methanol suspendiert und mittels Wasserstoff über Raney-Nickel bei einer Temperatur von 60 °C und einem Wasserstoffdruck von 60 bar reduziert. Nach Reaktionsende wird der Katalysator bei 70 °C durch Filtration entfernt und das Filtrat auf 20 °C abgekühlt. Das auskristallisierte Produkt wird abgesaugt. Es besitzt die Formel

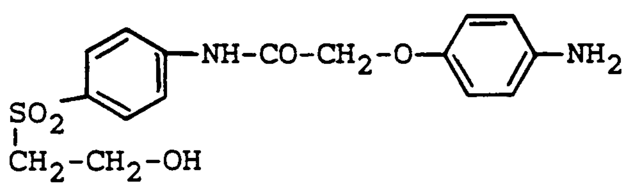

und hat folgende physikalische Daten:
Schmelzpunkt: 158 °C ;
Banden im IR-Spektrum:
3352 $cm^{-1}$ (NH) ; 3290 $cm^{-1}$ (NH) ; 2912 $cm^{-1}$ (CH) ; 1680 $cm^{-1}$ (CO) .

**Beispiel C**

30 Teile der Anilinverbindung von Beispiel B werden in 120 Teile Schwefelsäure-Monohydrat bei 10 °C eingetragen. Man erwärmt die erhaltene Suspension anschließend langsam auf 20 °C und rührt sie noch 16 Stunden weiter, gibt sie danach auf 1600 Teile Eis und saugt den Niederschlag ab.
Die Verbindung besitzt die Formel

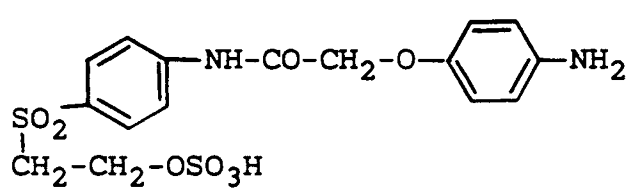

hat einen Schmelzpunkt von oberhalb 300 ° C und zeigt folgende Banden im IR-Spektrum:
3424 $cm^{-1}$ (NH) ; 3352 $cm^{-1}$ (NH) ; 2928 $cm^{-1}$ (CH) ; 1695 $cm^{-1}$ (CO) .

**Beispiel 1**

Eine wäßrige Lösung mit einem pH-Wert von 6,9 von 21,5 Teilen der Anilinverbindung von Beispiel C in 3000 Teilen Wasser wird mit 11 Teilen einer wäßrigen 5n-Natriumnitritlösung versetzt, und das Gemisch wird bei 5 ° C in 30 Teile einer wäßrigen konzentrierten Salzsäure eingerührt. Man rührt noch eine Stunde nach und entfernt überschüssiges Nitrit mit Amidosulfonsäure. Zu der Diazoniumsalz-Suspension gibt man bei 5 ° C und unter Einhaltung eines pH-Wertes von 6,5 15 Teile 6-Sulfo-2-acetylamino-8-naphthol hinzu und rührt den Ansatz anschließend noch etwa drei Stunden bei 15 bis 20 ° C nach, stellt den pH-Wert auf 4,5 bis 5 und isoliert die erfindungsgemäße Azoverbindung durch Aussalzen mit Natriumchlorid und Filtration.

Sie hat, in Form der freien Säure geschrieben, die Formel

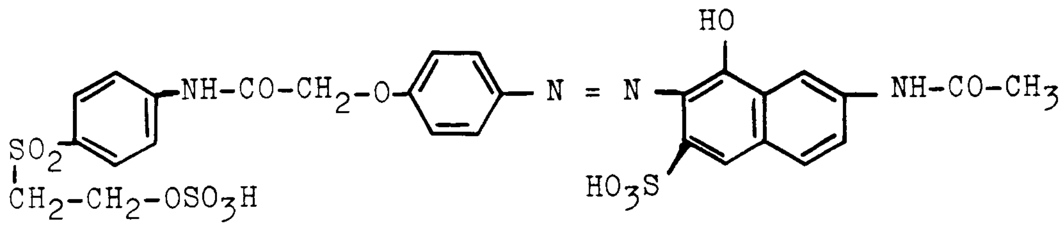

($\lambda_{max}$= 509 nm)

und besitzt sehr gute faserreaktive Farbstoffeigenschaften. Bei Anwendung der für faserreaktive Farbstoffe bekannten Applikations- und Fixierverfahren erhält man mit der erfindungsgemäßen Azoverbindung auf den in der Beschreibung genannten Materialien, wie insbesondere Cellulosefasermaterialien, beispielsweise Baumwolle, farbstarke, rote Färbungen und Drucke mit guten Echtheitseigenschaften.

**Beispiele 2 bis 55**

In den nachfolgenden Tabellenbeispielen sind weitere erfindungsgemäße Azoverbindungen entsprechend der in Form der freien Säure geschriebenen allgemeinen Formel (A)

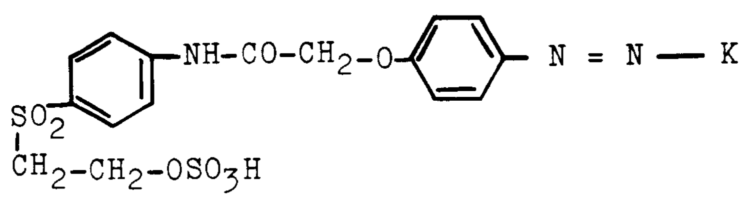

(A)

mit Hilfe ihrer Kupplungskomponente H-K beschrieben. Sie lassen sich in erfindungsgemäßer Weise, beispielsweise analog dem obigen Beispiel 1, herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften.

| Bsp. | Kupplungskomponente H-K in Formel (A) | Farbton |
|---|---|---|
| 2 | 6-Sulfo-3-benzoylamino-8-naphthol | rot |
| 3 | 1-(4'-ß-Sulfatoethylsulfonyl-phenyl)-3-methyl-5-pyrazolon | gelb (416) |
| 4 | 4,6-Disulfo-1-acetylamino-8-naphthol | blaustichig rot (546) |
| 5 | 3,6-Disulfo-2-amino-naphthalin | rot |
| 6 | 5,7-Disulfo-2-amino-naphthalin | marineblau |
| 7 | 4-Sulfo-1-naphthol | rot |
| 8 | 3,6-Disulfo-1-naphthol | rot |
| 9 | 1-(N-ß-Sulfoethyl)-4-methyl-2-hydroxy-6-pyridon | orange |
| 10 | 3,6,8-Trisulfo-2-amino-naphthalin | rot |
| 11 | 6-Sulfo-3-acetylamino-8-naphthol | rot (497) |
| 12 | N,N-Bis-(ß-sulfatoethyl)-anilin | orange |
| 13 | N-Ethyl-N-(ß-sulfatoethyl)-anilin | rot |
| 14 | 6-Sulfo-2-acetylamino-8-naphthol | rot (509) |
| 15 | 3,6-Disulfo-1-benzoylamino-8-naphthol | blaustichig rot (523) |
| 16 | 4,6-Disulfo-1-benzoylamino-8-naphthol | blaust. rot |
| 17 | N,N-Bis-(ß-sulfatoethyl)-3-chlor-anilin | orange |
| 18 | 5-Sulfo-1-naphthol | rot |

| Bsp. | Kupplungskomponente H-K in Formel (A) | Farbton |
|---|---|---|
| 19 | 3,6-Disulfo-1-amino-2-{2'-sulfo-5'-[5"-(ß-sulfatoethylsulfonyl)-1",2",3"-benzotriazol-1"-yl]-phenyl-diazoyl}-8-naphthol | grünstichig blau |
| 20 | 3,6-Disulfo-1-amino-2-[4'-(ß-sulfatoethylsulfonyl)-phenyl-diazoyl]-8-naphthol | grünstichig blau (625) |
| 21 | 6-Sulfo-2-methylamino-8-naphthol | rot |
| 22 | 4-Sulfo-diphenylamin | rot |
| 23 | 5-Sulfo-2-acetylamino-7-naphthol | rot |
| 24 | 3,6-Disulfo-2-acetylamino-8-naphthol | rot |
| 25 | 2,4-Disulfo-1-amino-8-naphthol | blau |
| 26 | 3,6-Disulfo-1-amino-8-naphthol (in 7-Stellung gekuppelt) | blau |
| 27 | 4,6-Disulfo-1-amino-8-naphthol (in 7-Stellung gekuppelt) | blau |
| 28 | 3,6-Disulfo-1-phenylureido-8-naphthol | blau |
| 29 | 3-Sulfo-1-naphthol | rot |
| 30 | 5-Sulfo-2-naphthol | rot |
| 31 | 6-Sulfo-2-naphthol | blaust. rot |
| 32 | 8-Sulfo-2-naphthol | blaust. rot |
| 33 | 3,6-Disulfo-1-acetylamino-8-naphthol | blau |
| 34 | N,N-Bis-(ß-hydroxyethyl)-anilin | rot |
| 35 | 3,6,8-Trisulfo-1-naphthol | blaust. rot |
| 36 | 3,6-Disulfo-2-naphthol | rot |
| 37 | 3,6-Disulfo-1-acetylamino-8-naphthol | blau |
| 38 | 4,6-Disulfo-1-acetylamino-8-naphthol | blau |
| 39 | N-Ethyl-N-(3'-sulfobenzyl)-anilin | rot |
| 40 | 5-Sulfo-1,4-dimethyl-2-hydroxy-6-pyridon | gelb |
| 41 | 2-Carboxy-acetoacetylanilin | gelb |
| 42 | 5-Sulfo-8-hydroxy-chinolin | rot |
| 43 | 3-Sulfo-acetoacetylanilin | gelb |
| 44 | 1-(4'-Sulfophenyl)-3-methyl-5-pyrazolon | gelb (413) |
| 45 | 1-(2'-Chlor-5'-sulfophenyl)-3-methyl-5-pyrazolon | gelb |
| 46 | 1-(2',5'-Disulfophenyl)-3-methyl-5-pyrazolon | gelb |
| 47 | 1-(4',8'-Disulfo-naphth-2'-yl)-3-methyl-5-pyrazolon | gelb |

| Bsp. | Kupplungskomponente H-K in Formel (A) | Farbton |
|---|---|---|
| 48 | 1-(4'-Sulfophenyl)-3-methyl-5-amino-pyrazol | gelb |
| 49 | 3,6-Disulfo-1-[4'-chlor-6'-(3"-sulfo-phenyl)-amino-1',3',5'-triazin-2'-yl]-amino-8-naphthol | blaustichig rot |
| 50 | 3,6-Disulfo-1-{4'-chlor-6'-[4"-(ß-sulfatoethylsulfonyl)-phenyl]-amino-1',3',5'-triazin-2'-yl}-amino-8-naphthol | blaustichig rot (529) |
| 51 | 6,8-Disulfo-2-naphthol | rot |
| 52 | 6-Sulfo-2-benzoylamino-8-naphthol | rot |
| 53 | N-Acetoacetyl-(2-methoxy-5-methyl-4-sulfo)-anilin | gelb |
| 54 | 1-(4'-Sulfophenyl)-3-carboxy-5-pyrazolon | gelb |
| 55 | 5-Acetylamino-2-sulfo-anilin | gelb |

**Beispiele 56 bis 109**

In den nachfolgenden Tabellenbeispielen sind weitere erfindungsgemäße Azoverbindungen entsprechend der in Form der freien Säure geschriebenen allgemeinen Formel (B)

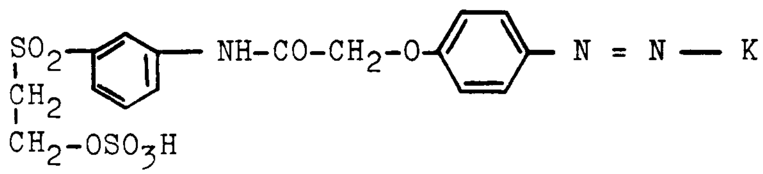

(B)

mit Hilfe ihrer Kupplungskomponente H-K beschrieben. Sie lassen sich in erfindungsgemäßer Weise, beispielsweise analog dem obigen Beispiel 1, herstellen und besitzen ebenfalls sehr gute faserreaktive Farbstoffeigenschaften. Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften.

| Bsp. | Kupplungskomponente H-K in Formel (B) | Farbton |
|---|---|---|
| 56 | 6-Sulfo-3-benzoylamino-8-naphthol | rot |
| 57 | 1-(4'-ß-Sulfatoethylsulfonyl-phenyl)-3-methyl-5-pyrazolon | gelb (410) |
| 58 | 4,6-Disulfo-1-acetylamino-8-naphthol | blaustichig rot (508) |
| 59 | 3,6-Disulfo-2-amino-naphthalin | rot |
| 60 | 5,7-Disulfo-2-amino-naphthalin | marineblau |
| 61 | 4-Sulfo-1-naphthol | rot |
| 62 | 3,6-Disulfo-1-naphthol | rot |
| 63 | 1-(N-ß-Sulfoethyl)-4-methyl-2-hydroxy-6-pyridon | orange |
| 64 | 3,6,8-Trisulfo-2-amino-naphthalin | rot |
| 65 | 6-Sulfo-3-acetylamino-8-naphthol | rot (498) |
| 66 | N,N-Bis-(ß-sulfatoethyl)-anilin | orange |
| 67 | N-Ethyl-N-(ß-sulfatoethyl)-anilin | rot |
| 68 | 6-Sulfo-2-acetylamino-8-naphthol | rot (509) |
| 69 | 3,6-Disulfo-1-benzoylamino-8-naphthol | blaustichig rot (523) |
| 70 | 4,6-Disulfo-1-benzoylamino-8-naphthol | blaust. rot |
| 71 | N,N-Bis-(ß-sulfatoethyl)-3-chlor-anilin | orange |
| 72 | 5-Sulfo-1-naphthol | rot |
| 73 | 3,6-Disulfo-1-amino-2-{2'-sulfo-5'-[5"-(ß-sulfatoethylsulfonyl)-1",2",3"-benzotriazol-1"-yl]-phenyl-diazoyl}-8-naphthol | grünstichig blau |
| 74 | 3,6-Disulfo-1-amino-2-[4'-(ß-sulfato-ethylsulfonyl)-phenyl-diazoyl]-8-naphthol | grünstichig blau (625) |
| 75 | 6-Sulfo-2-methylamino-8-naphthol | rot |
| 76 | 4-Sulfo-diphenylamin | rot |
| 77 | 5-Sulfo-2-acetylamino-7-naphthol | rot |
| 78 | 3,6-Disulfo-2-acetylamino-8-naphthol | rot |
| 79 | 2,4-Disulfo-1-amino-8-naphthol | blau |
| 80 | 3,6-Disulfo-1-amino-8-naphthol (in 7-Stellung gekuppelt) | blau |
| 81 | 4,6-Disulfo-1-amino-8-naphthol (in 7-Stellung gekuppelt) | blau |
| 82 | 3,6-Disulfo-1-phenylureido-8-naphthol | blau |

| Bsp. | Kupplungskomponente H-K in Formel (B) | Farbton |
|---|---|---|
| 83 | 3-Sulfo-1-naphthol | rot |
| 84 | 5-Sulfo-2-naphthol | rot |
| 85 | 6-Sulfo-2-naphthol | blaust. rot |
| 86 | 8-Sulfo-2-naphthol | blaust. rot |
| 87 | 3,6-Disulfo-1-acetylamino-8-naphthol | blau |
| 88 | N,N-Bis-(ß-hydroxyethyl)-anilin | rot |
| 89 | 3,6,8-Trisulfo-1-naphthol | blaust. rot |
| 90 | 3,6-Disulfo-2-naphthol | rot |
| 91 | 3,6-Disulfo-1-acetylamino-8-naphthol | blau |
| 92 | 4,6-Disulfo-1-acetylamino-8-naphthol | blau |
| 93 | N-Ethyl-N-(3'-sulfobenzyl)-anilin | rot |
| 94 | 5-Sulfo-1,4-dimethyl-2-hydroxy-6-pyridon | gelb |
| 95 | 2-Carboxy-acetoacetylanilin | gelb |
| 96 | 5-Sulfo-8-hydroxy-chinolin | rot |
| 97 | 3-Sulfo-acetoacetylanilin | gelb |
| 98 | 1-(4'-Sulfophenyl)-3-methyl-5-pyrazolon | gelb (414) |
| 99 | 1-(2'-Chlor-5'-sulfophenyl)-3-methyl-5-pyrazolon | gelb |
| 100 | 1-(2',5'-Disulfophenyl)-3-methyl-5-pyrazolon | gelb |
| 101 | 1-(4',8'-Disulfo-naphth-2'-yl)-3-methyl-5-pyrazolon | gelb |
| 102 | 1-(4'-Sulfophenyl)-3-methyl-5-amino-pyrazol | gelb |
| 103 | 3,6-Disulfo-1-[4'-chlor-6'-(3"-sulfophenyl)-amino-1',3',5'-triazin-2'-yl]-amino-8-naphthol | blaustichig rot |
| 104 | 3,6-Disulfo-1-{4'-chlor-6'-[4"-(ß-sulfatoethylsulfonyl)-phenyl]-amino-1',3',5'-triazin-2'-yl}-amino-8-naphthol | blaustichig rot (529) |
| 105 | 6,8-Disulfo-2-naphthol | rot |
| 106 | 6-Sulfo-2-benzoylamino-8-naphthol | rot |
| 107 | N-Acetoacetyl-(2-methoxy-5-methyl-4-sulfo)-anilin | gelb |
| 108 | 1-(4'-Sulfophenyl)-3-carboxy-5-pyrazolon | gelb |
| 109 | 5-Acetylamino-2-sulfo-anilin | gelb |

## Patentansprüche

## Patentansprüche für folgende Vertragsstaaten : BE, CH, DE, FR, GB, IT, LI

1. Eine wasserlösliche Azoverbindung entsprechend der allgemeinen Formel (1)

   $$D - N = N - K \quad (1)$$

   in welcher bedeuten:
   D ist eine Gruppe der allgemeinen Formel (2)

   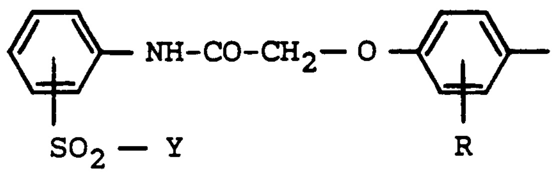

   (2)

   in welcher
   Y Vinyl oder eine Gruppe der allgemeinen Formel (3)

   $$-CH_2-CH_2-X \quad (3)$$

   ist, in welcher
   X ein Substituent ist, der durch ein Alkali unter Bildung der Vinylgruppe eliminierbar ist, und
   R ein Wasserstoff, Nitro, Alkyl von 1 bis 4 C-Atomen, Alkoxy von 1 bis 4 C-Atomen, Carboxy, Hydroxy oder Halogen ist;
   K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure oder einer aromatischen Sulfonsäure oder einer N-substituierten Carbaminsäure oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine oder mehrere faserreaktive Gruppen enthalten kann.

2. Verbindung nach Anspruch 1, dadurch gekennzeichnet, daß K ein Rest der allgemeinen Formel

   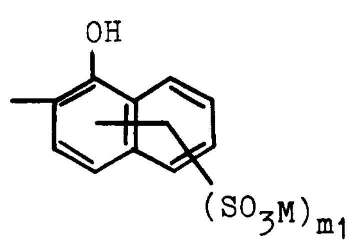

   ist, in welcher $m_1$ für die Zahl 1, 2 oder 3 steht und M ein Wasserstoffatom oder ein Alkalimetall ist.

**3.** Verbindung nach Anspruch 1, dadurch gekennzeichnet, daß K ein Rest der allgemeinen Formel

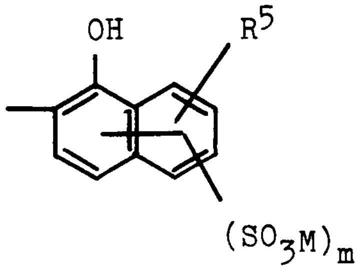

ist, in welcher $R^5$ Benzoylamino oder Alkanoylamino von 2 bis 5 C-Atomen ist, m die Zahl 1 oder 2 ist und M Wasserstoff oder ein Alkalimetall ist.

**4.** Verbindung nach Anspruch 1, dadurch gekennzeichnet, daß K ein Rest der allgemeinen Formel

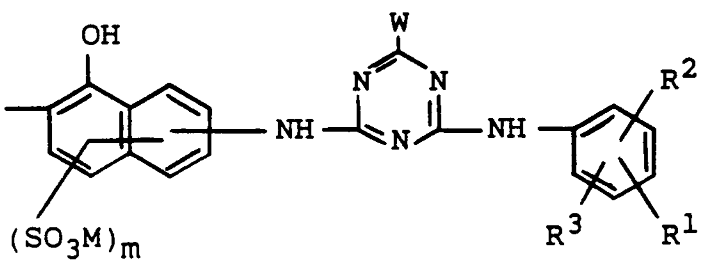

(4v)

ist, in welcher m die Zahl 1 oder 2 ist, W Sulfo, Alkylsulfonyl von 1 bis 4 C-Atomen, Phenylsulfonyl, Brom, Fluor oder Chlor ist, $R^1$ die Gruppe $-SO_2-Y$ mit einer der in Anspruch 1 genannten Bedeutungen ist, $R^2$ Wasserstoff, Alkyl von 1 bis 4 C-Atomen, Alkoxy von 1 bis 4 C-Atomen, Chlor, Brom, Carboxy, Sulfo oder Nitro ist, $R^3$ Wasserstoff ist und M Wasserstoff oder ein Alkalimetall ist.

**5.** Verbindung nach Anspruch 1, dadurch gekennzeichnet, daß K ein Rest der allgemeinen Formel

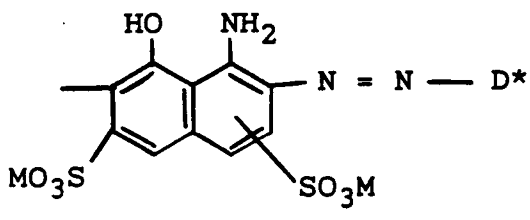

ist, in welcher D* Phenyl ist, das durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der allgemeinen Formel $-SO_2-Y$ mit Y einer in Anspruch 1 genannten Bedeutung substituiert sein kann, oder Naphthyl ist, das durch 1, 2 oder 3 Sulfogruppen oder durch 1 oder 2 Sulfogruppen und 1 oder 2 Gruppen der allgemeinen Formel $-SO_2-Y$ mit Y einer in Anspruch 1 genannten Bedeutung oder nur durch eine solche Gruppe $-SO_2-Y$ substituiert ist, und M ein Wasserstoffatom oder ein Alkalimetall ist.

**6.** Verbindung nach Anspruch 4, dadurch gekennzeichnet, daß W Fluor oder Chlor ist.

**7.** Verbindung nach Anspruch 5, dadurch gekennzeichnet, daß D* Phenyl ist, das in para-Stellung zur Azogruppe durch eine Gruppe der allgemeinen Formel $-SO_2-Y$ mit Y der in Anspruch 1 genannten Bedeutung substituiert ist.

**8.** Verbindung nach mindestens einem der Ansprüche 1 bis 7, dadurch gekennzeichnet, daß R Wasserstoff ist.

**9.** Verbindung nach mindestens einem der Ansprüche 1 bis 8, dadurch gekennzeichnet, daß Y Vinyl oder β-Sulfatoethyl ist.

**10.** Verbindung nach mindestens einem der Ansprüche 1 bis 9, dadurch gekennzeichnet, daß Y β-Sulfatoethyl ist.

**11.** Verfahren zur Herstellung einer Verbindung der in Anspruch 1 genannten und definierten allgemeinen Formel (1), dadurch gekennzeichnet, daß man eine Diazoniumverbindung einer Aminoverbindung der allgemeinen Formel (5)

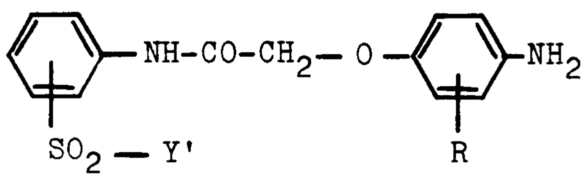

(5)

in welcher Y′ eine der Bedeutungen von Y hat oder die β-Hydroxyethyl-Gruppe ist und R die in Anspruch 1 genannte Bedeutung besitzt, mit einer Kupplungskomponente der allgemeinen Formel H-K mit K der in Anspruch 1 genannten Bedeutung kuppelt und gegebenenfalls die gebildete Azoverbindung mit einer weiteren Diazoverbindung oder Kupplungskomponente umsetzt und im Falle, daß Y′ die β-Hydroxyethyl-Gruppe ist, diese in der gebildeten Azoverbindung in eine Gruppe Y der in Anspruch 1 genannten Bedeutung überführt.

**12.** Verwendung einer Verbindung entsprechend der allgemeinen Formel (1) von Anspruch 1 zum Färben (einschließlich Bedrucken) von hydroxy- und/oder carbonamidgruppenhaltigem Material, insbesondere Fasermaterial.

**13.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine Verbindung entsprechend der allgemeinen Formel (1) von Anspruch 1 einsetzt.

**14.** Eine Verbindung entsprechend der allgemeinen Formel (8)

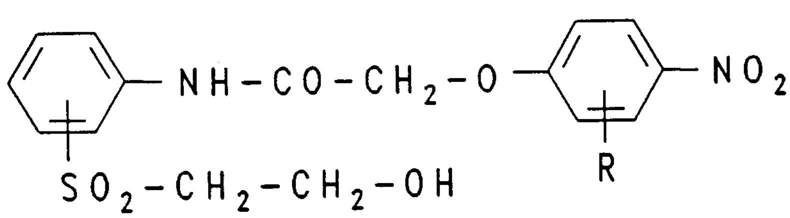

(8)

in welcher R Wasserstoff, Nitro, Alkyl von 1 bis 4 C-Atomen, Alkoxy von 1 bis 4 C-Atomen, Carboxy, Hydroxy oder Halogen ist.

**15.** Eine Verbindung entsprechend der allgemeinen Formel

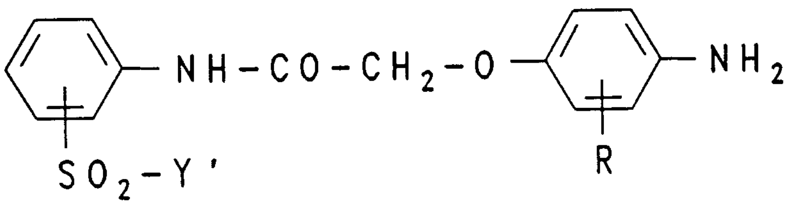

(5)

in welcher

R Wasserstoff, Nitro, Alkyl von 1 bis 4 C-Atomen, Alkoxy von 1 bis 4 C-Atomen, Carboxy, Hydroxy oder Halogen ist und

Y' $\beta$-Hydroxyethyl oder Vinyl ist oder Ethyl ist, das in $\beta$-Stellung einen Substituenten enthält, der durch ein Alkali unter Bildung der Vinylgruppe eliminierbar ist.

**16.** Eine Verbindung nach Anspruch 15, dadurch gekennzeichnet, daß Y' $\beta$-Hydroxyethyl, $\beta$-Sulfatoethyl oder Vinyl ist.

**17.** Eine Verbindung nach Anspruch 15 oder 16, dadurch gekennzeichnet, daß R Wasserstoff ist.

**18.** Verfahren zur Herstellung einer Verbindung von Anspruch 14 oder 15, dadurch gekennzeichnet, daß man eine Verbindung der allgemeinen Formel (6)

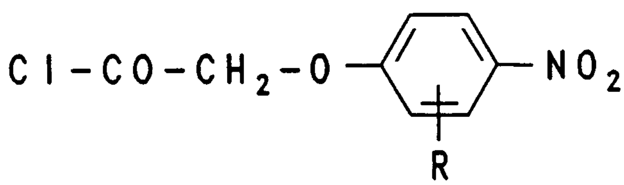

(6)

in welcher R die in Anspruch 14 genannte Bedeutung hat, mit einer Aminoverbindung der allgemeinen Formel (7)

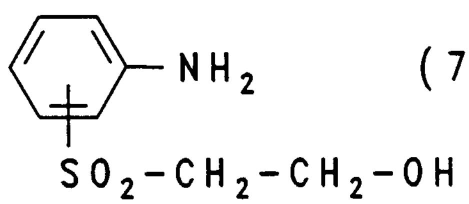

(7)

umsetzt und in der so erhaltenen Verbindung der allgemeinen Formel (8) die Nitrogruppe zur Verbindung der allgemeinen Formel (5A)

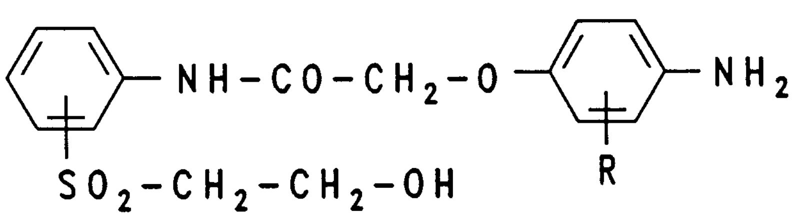

(5A)

mit R der obengenannten Bedeutung reduziert und gegebenenfalls in der Verbindung der Formel (5A) die $\beta$-Hydroxyethylsulfonyl-Gruppe in eine Gruppe der Formel Y-$SO_2$- mit Y einer der in Anspruch 1 genannten Bedeutungen überführt.

**19.** Verwendung einer Verbindung von Anspruch 14 oder 15 zur Synthese von Farbstoffen, insbesondere von Azofarbstoffen.

**Patentansprüche für folgenden Vertragsstaat : ES**

**1.** Verfahren zur Herstellung einer wasserlöslichen Azoverbindung entsprechend der allgemeinen Formel (1)

D — N = N — K (1)

in welcher bedeuten:

D ist eine Gruppe der allgemeinen Formel (2)

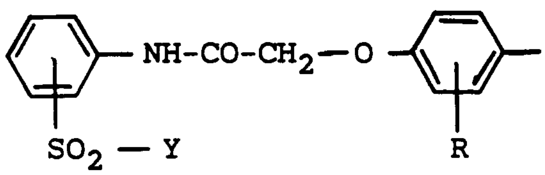

(2)

in welcher

Y Vinyl oder eine Gruppe der allgemeinen Formel (3)

$— CH_2 — CH_2 — X$ (3)

ist, in welcher

X ein Substituent ist, der durch ein Alkali unter Bildung der Vinylgruppe eliminierbar ist, und

R ein Wasserstoff, Nitro, Alkyl von 1 bis 4 C-Atomen, Alkoxy von 1 bis 4 C-Atomen, Carboxy, Hydroxy oder Halogen ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure oder einer aromatischen Sulfonsäure oder einer N-substituierten Carbaminsäure oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine oder mehrere faserreaktive Gruppen enthalten kann,

dadurch gekennzeichnet, daß man eine Diazoniumverbindung einer Aminoverbindung der allgemeinen Formel (5)

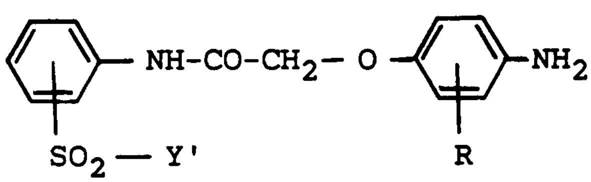

(5)

in welcher Y′ eine der Bedeutungen von Y hat oder die β-Hydroxyethyl-Gruppe ist und R die oben genannte Bedeutung besitzt, mit einer Kupplungskomponente der allgemeinen Formel H-K mit K der oben genannten Bedeutung kuppelt und gegebenenfalls die gebildete Azoverbindung mit einer weiteren Diazoverbindung oder Kupplungskomponente umsetzt und im Falle, daß Y′ die β-Hydroxyethyl-Gruppe ist, diese in der gebildeten Azoverbindung in eine Gruppe Y der oben genannten Bedeutung überführt.

2. Verfahren nach Anspruch 1, dadurch gekennzeichnet, daß K ein Rest der allgemeinen Formel

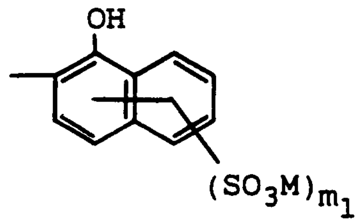

ist, in welcher $m_1$ für die Zahl 1, 2 oder 3 steht und M ein Wasserstoffatom oder ein Alkalimetall ist.

3. Verfahren nach Anspruch 1, dadurch gekennzeichnet, daß K ein Rest der allgemeinen Formel

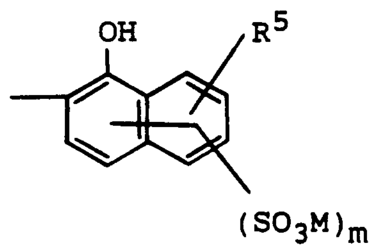

ist, in welcher $R^5$ Benzoylamino oder Alkanoylamino von 2 bis 5 C-Atomen ist, m die Zahl 1 oder 2 ist und M Wasserstoff oder ein Alkalimetall ist.

4. Verfahren nach Anspruch 1, dadurch gekennzeichnet, daß K ein Rest der allgemeinen Formel

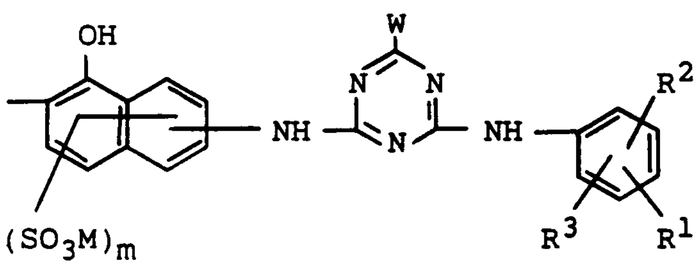

(4v)

ist, in welcher m die Zahl 1 oder 2 ist, W Sulfo, Alkylsulfonyl von 1 bis 4 C-Atomen, Phenylsulfonyl, Brom, Fluor oder Chlor ist, $R^1$ die Gruppe $-SO_2-Y$ mit einer der in Anspruch 1 genannten Bedeutungen ist, $R^2$ Wasserstoff, Alkyl von 1 bis 4 C-Atomen, Alkoxy von 1 bis 4 C-Atomen, Chlor, Brom, Carboxy, Sulfo oder Nitro ist, $R^3$ Wasserstoff ist und M Wasserstoff oder ein Alkalimetall ist.

5. Verfahren nach Anspruch 1, dadurch gekennzeichnet, daß K ein Rest der allgemeinen Formel

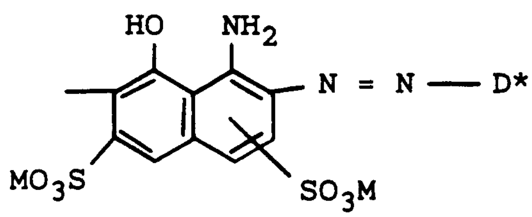

ist, in welcher D* Phenyl ist, das durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der allgemeinen Formel $-SO_2-Y$

mit Y einer in Anspruch 1 genannten Bedeutung substituiert sein kann, oder Naphthyl ist, das durch 1, 2 oder 3 Sulfogruppen oder durch 1 oder 2 Sulfogruppen und 1 oder 2 Gruppen der allgemeinen Formel $-SO_2-Y$ mit Y einer in Anspruch 1 genannten Bedeutung oder nur durch eine solche Gruppe $-SO_2-Y$ substituiert ist, und M ein Wasserstoffatom oder ein Alkalimetall ist.

**6.** Verfahren nach Anspruch 4, dadurch gekennzeichnet, daß W Fluor oder Chlor ist.

**7.** Verfahren nach Anspruch 5, dadurch gekennzeichnet, daß D* Phenyl ist, das in para-Stellung zur Azogruppe durch eine Gruppe der allgemeinen Formel $-SO_2-Y$ mit Y der in Anspruch 1 genannten Bedeutung substituiert ist.

**8.** Verfahren nach mindestens einem der Ansprüche 1 bis 7, dadurch gekennzeichnet, daß R Wasserstoff ist.

**9.** Verfahren nach mindestens einem der Ansprüche 1 bis 8, dadurch gekennzeichnet, daß Y Vinyl oder β-Sulfatoethyl ist.

**10.** Verfahren nach mindestens einem der Ansprüche 1 bis 9, dadurch gekennzeichnet, daß Y β-Sulfatoethyl ist.

**11.** Verwendung einer Verbindung entsprechend der allgemeinen Formel (1) von Anspruch 1 zum Färben (einschließlich Bedrucken) von hydroxy- und/oder carbonamidgruppenhaltigem Material, insbesondere Fasermaterial.

**12.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine Verbindung entsprechend der allgemeinen Formel (1) von Anspruch 1 einsetzt.

**13.** Verfahren zur Herstellung einer Verbindung entsprechend der allgemeinen Formel (8) oder (5)

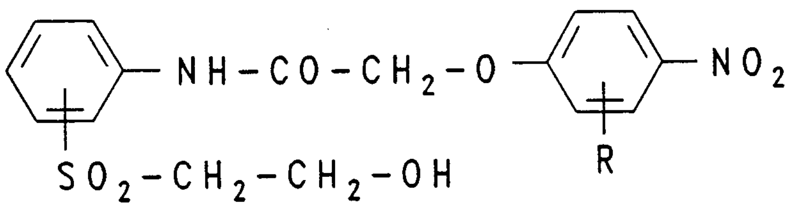

(8)

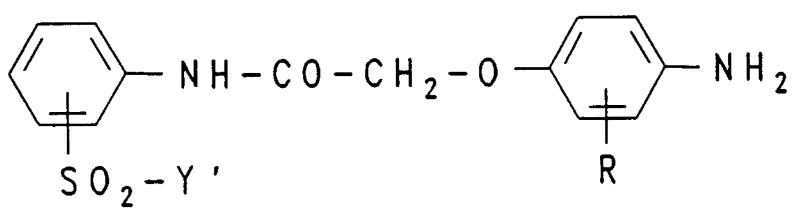

(5)

in welchen

R Wasserstoff, Nitro, Alkyl von 1 bis 4 C-Atomen, Alkoxy von 1 bis 4 C-Atomen, Carboxy, Hydroxy oder Halogen ist und

Y' β-Hydroxyethyl oder Vinyl ist oder Ethyl ist, das in β-Stellung einen Substituenten enthält, der durch ein Alkali unter Bildung der Vinylgruppe eliminierbar ist,

dadurch gekennzeichnet, daß man eine Verbindung der allgemeinen Formel (6)

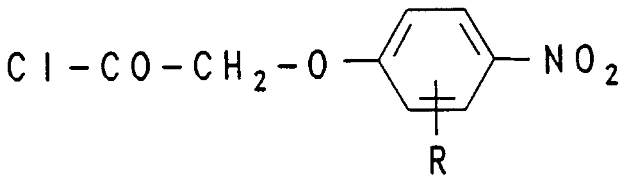

(6)

in welcher R die oben genannte Bedeutung hat, mit einer Aminoverbindung der allgemeinen Formel

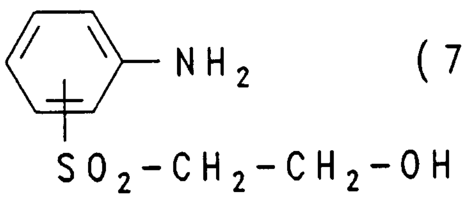

(7)

umsetzt und in der so erhaltenen Verbindung der allgemeinen Formel (8) die Nitrogruppe zur Verbindung der allgemeinen Formel (5A)

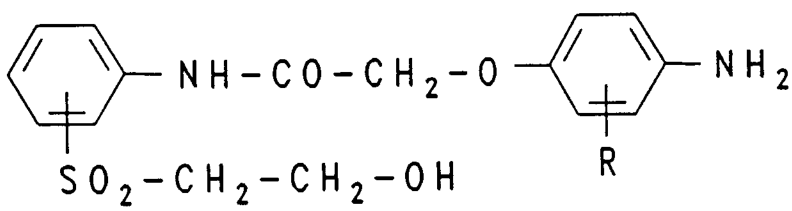

(5A)

mit R der obengenannten Bedeutung reduziert und gegebenenfalls in der Verbindung der Formel (5A) die β-Hydroxyethylsulfonyl-Gruppe in eine Gruppe der Formel Y-$SO_2$- überführt, in welcher Y Vinyl oder Ethyl ist, das in β-Stellung einen Substituenten enthält, der durch ein Alkali unter Bildung der Vinylgruppe eliminierbar ist.

**14.** Verfahren nach Anspruch 13, dadurch gekennzeichnet, daß Y' β-Hydroxyethyl, β-Sulfatoethyl oder Vinyl ist.

**15.** Verfahren nach Anspruch 13 oder 14, dadurch gekennzeichnet, daß R Wasserstoff ist.

**Claims**
**Claims for the following Contracting States : BE, CH, DE, FR, GB, IT, LI**

**1.** A water-soluble azo compound of the formula (1)

D — N = N — K (1)

in which the symbols have the following meanings:
D is a group of the general formula (2)

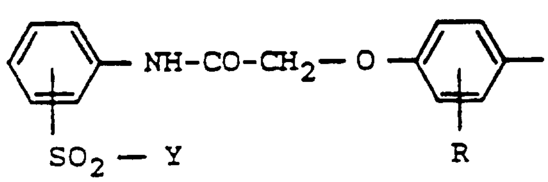

(2)

in which
Y is vinyl or a group of the formula (3)

$$-CH_2-CH_2-X \quad (3)$$

in which

X is a substituent which can be eliminated by an alkali with the formation of the vinyl group, and

R is hydrogen, nitro, alkyl of 1 to 4 carbon atoms, alkoxy of 1 to 4 carbon atoms, carboxyl, hydroxyl or halogen,

K is a radical of a mono-coupleable water-soluble coupling component, which can additionally contain an azo group, or the radical of a doubly-coupleable water-soluble coupling component, each from the series of aminobenzenes, phenols, in particular their sulfonic acids and carboxylic acids, naphthols, in particular their sulfonic acids and carboxylic acids, aminonaphthols, in particular their sulfonic acids, acylaminonaphthols, in particular their sulfonic acids, with the acyl radical of an alkane- or alkenecarboxylic acid each having 1 to 4 or 2 to 4 carbon atoms in the alkyl or alkenyl radical, or of an aromatic carboxylic acid, or of an aromatic sulfonic acid, or of an N-substituted carbamic acid, or from the series of dihydroxynaphthalenesulfonic acids, phenylazo- and naphthylazoaminonaphtholsulfonic acids, 5-pyrazolones and 5-aminopyrazoles, acetoacetylarylides, 2-hydroxy-6-pyridones and hydroxyquinolines, it also being possible for K to contain one or more fiber-reactive groups in addition to the substituents customary in dyes.

**2.** A compound as claimed in claim 1, wherein K is a radical of the formula

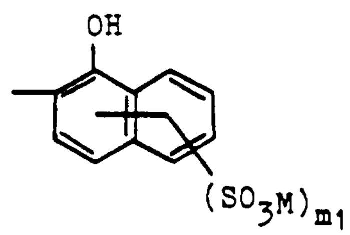

in which $m_1$ is the number 1, 2 or 3 and M is a hydrogen atom or an alkali metal.

**3.** A compound as claimed in claim 1, wherein K is a radical of the formula

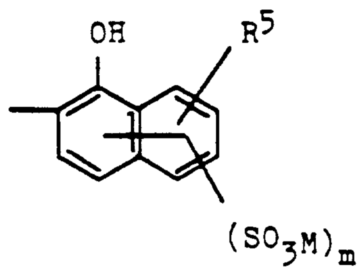

in which $R^5$ is benzoylamino or alkanoylamino of 2 to 5 carbon atoms, m is the number 1 or 2 and M is hydrogen or an alkali metal.

4. A compound as claimed in claim 1, wherein K is a radical of the formula

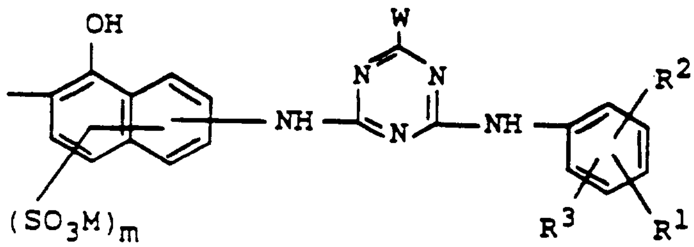

(4v)

in which m is the number 1 or 2, W is sulfo, alkylsulfonyl of 1 to 4 carbon atoms, phenylsulfonyl, bromine, fluorine or chlorine, $R^1$ is the group $-SO_2-Y$ having one of the meanings mentioned in claim 1, $R^2$ is hydrogen, alkyl of 1 to 4 carbon atoms, alkoxy of 1 to 4 carbon atoms, chlorine, bromine, carboxyl, sulfo or nitro, $R^3$ is hydrogen and M is hydrogen or an alkali metal.

5. A compound as claimed in claim 1, wherein K is a radical of the formula

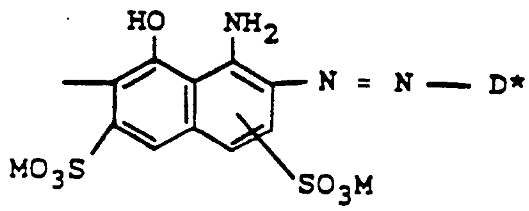

in which D* is phenyl, which can be substituted by 1, 2 or 3 substituents from the group comprising alkyl of 1 to 4 carbon atoms, alkoxy of 1 to 4 carbon atoms, chlorine, bromine, hydroxyl, carboxyl, sulfo, carbamoyl, sulfamoyl and alkanoylamino of 2 to 5 carbon atoms and/or by a group of the formula $-SO_2-Y$ where Y has one of the meanings mentioned in claim 1, or is naphthyl which is substituted by 1, 2 or 3 sulfo groups or by 1 or 2 sulfo groups and 1 or 2 groups of the formula $-SO_2-Y$ where Y has one of the meanings mentioned in claim 1, or only by one of these groups $-SO_2-Y$, and M is a hydrogen atom or an alkali metal.

6. A compound as claimed in claim 4, wherein W is fluorine or chlorine.

7. A compound as claimed in claim 5, wherein D* is phenyl which is substituted in the para position relative to the azo group by a group of the formula $-SO_2-Y$ where Y has the meaning mentioned in claim 1.

8. A compound as claimed in at least one of claims 1 to 7, wherein R is hydrogen.

9. A compound as claimed in at least one of claims 1 to 8, wherein Y is vinyl or $\beta$-sulfatoethyl.

10. A compound as claimed in at least one of claims 1 to 9, wherein Y is $\beta$-sulfatoethyl.

11. A process for the preparation of a compound of the formula (1) mentioned and defined in claim 1, which comprises coupling a diazonium compound of an amino compound of the formula (5)

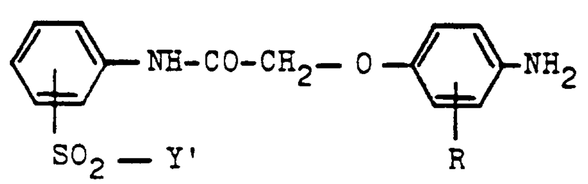

(5)

in which Y' has one of the meanings of Y or is a $\beta$-hydroxyethyl group and R has the meaning

mentioned in claim 1, with a coupling component of the formula H-K where K has the meaning mentioned in claim 1 and, if desired, reacting the resulting azo compound with a further diazo compound or coupling component and, in the case that Y' is a $\beta$-hydroxyethyl group, converting this group in the azo compound formed into a group Y of the meaning mentioned in claim 1.

**12.** The use of a compound of the formula (1) of claim 1 for the dyeing (including printing) of hydroxyl- and/or carboxamido-containing material, in particular fiber material.

**13.** A process for the dyeing (including printing) of hydroxyl- and/or carboxamido-containing material, in particular fiber material, in which a dye is applied to the material or incorporated therein and fixed by means of heat and/or by means of an alkaline agent, wherein the dye used is a compound of the formula (1) of claim 1.

**14.** A compound of the formula (8)

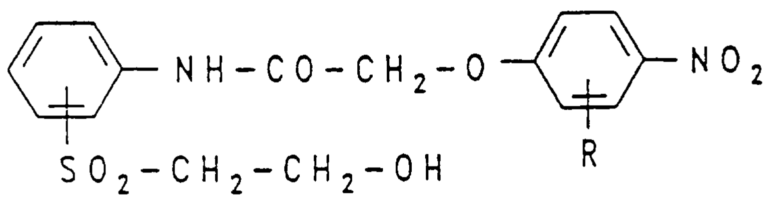

(8)

in which R is hydrogen, nitro, alkyl of 1 to 4 carbon atoms, alkoxy of 1 to 4 carbon atoms, carboxyl, hydroxyl or halogen.

**15.** A compound of the formula

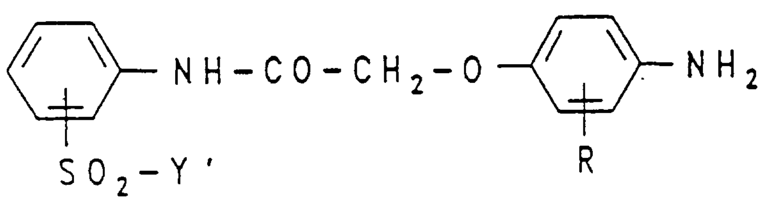

(5)

in which

R is hydrogen, nitro, alkyl of 1 to 4 carbon atoms, alkoxy of 1 to 4 carbon atoms, carboxyl, hydroxyl or halogen and

Y' is $\beta$-hydroxyethyl or vinyl or ethyl containing a substituent in the $\beta$-position which can be eliminated by an alkali with the formation of the vinyl group.

**16.** A compound as claimed in claim 15, wherein Y' is $\beta$-hydroxyethyl, $\beta$-sulfatoethyl or vinyl.

**17.** A compound as claimed in claim 15 or 16, wherein R is hydrogen.

**18.** A process for the preparation of a compound of claim 14 or 15, which comprises reacting a compound of the formula (6)

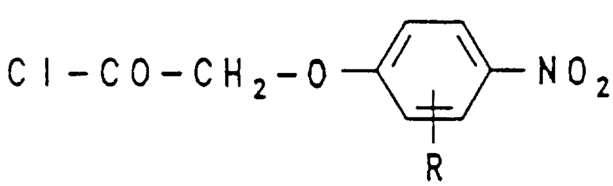

(6)

in which R has the meaning mentioned in claim 14 with an amino compound of the formula

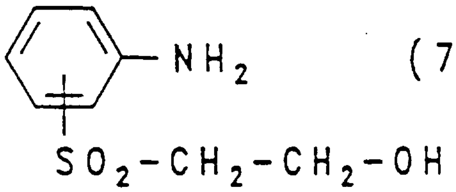 (7)

and reducing the nitro group in the compound of the formula (8) thus obtained to give the compound of the formula (5A)

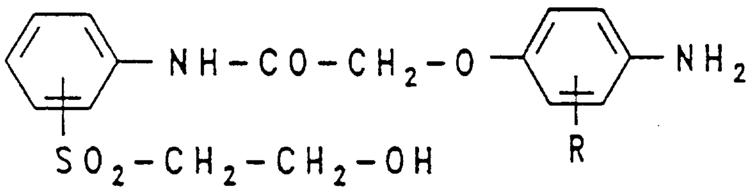 (5A)

where R has the abovementioned meaning and, if desired, converting the $\beta$-hydroxyethylsulfonyl group in the compound of the formula (5A) into a group of the formula Y-$SO_2$- where Y has one of the meanings mentioned in claim 1.

**19.** Use of a compound of claim 14 or 15 for the synthesis of dyes, in particular of azo dyes.

**Claims for the following Contracting State : ES**

**1.** A process for the preparation of a water-soluble azo compound of the formula (1)

D — N = N — K (1)

in which the symbols have the following meanings:

D is a group of the general formula (2)

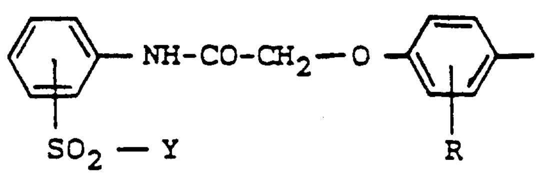 (2)

in which

Y is vinyl or a group of the formula (3)

$-CH_2-CH_2-X$ (3)

in which

X is a substituent which can be eliminated by an alkali with the formation of the vinyl group, and

R is hydrogen, nitro, alkyl of 1 to 4 carbon atoms, alkoxy of 1 to 4 carbon atoms, carboxyl, hydroxyl or halogen,

K is a radical of a mono-coupleable water-soluble coupling component, which can additionally contain an azo group, or the radical of a doubly-coupleable water-soluble coupling component, each from the series of aminobenzenes, phenols, in particular their sulfonic acids and carboxylic acids, naphthols, in particular their sulfonic acids and carboxylic acids, aminonaphthols, in particular their sulfonic acids, acylaminonaphthols, in particular their sulfonic acids,

with the acyl radical of an alkane- or alkenecarboxylic acid each having 1 to 4 or 2 to 4 carbon atoms in the alkyl or alkenyl radical or of an aromatic carboxylic acid, or of an aromatic sulfonic acid, or of an N-substituted carbamic acid, or from the series of dihydroxynaphthalenesulfonic acids, phenylazo- and naphthylazoaminonaphtholsulfonic acids, 5-pyrazolones and 5-aminopyrazoles, acetoacetylarylides, 2-hydroxy-6-pyridones and hydroxyquinolines, it also being possible for K to contain one or more fiber-reactive groups in addition to the substituents customary in dyes, which comprises coupling a diazonium compound of an amino compound of the general formula (5)

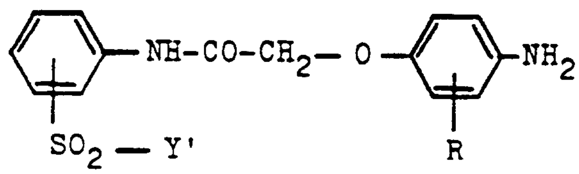

(5)

in which Y' has one of the meanings of Y or is a β-hydroxyethyl group and R has the meaning mentioned above, with a coupling component of the formula H-K where K has the meaning mentioned above and, if desired, reacting the resulting azo compound with a further diazo compound or coupling component and, in the case that Y' is a β-hydroxyethyl group, converting this group in the azo compound formed into a group Y of the meaning mentioned above.

**2.** The process as claimed in claim 1, wherein K is a radical of the formula

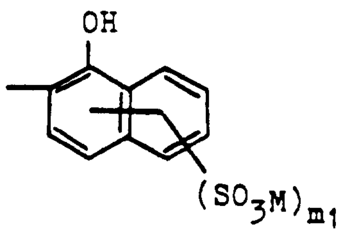

in which $m_1$ is the number 1, 2 or 3 and M is a hydrogen atom or an alkali metal.

**3.** The process as claimed in claim 1, wherein K is a radical of the formula

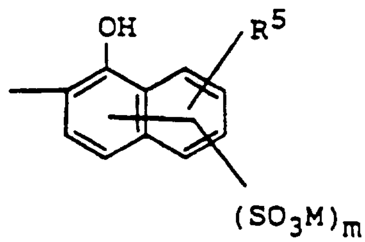

in which $R^5$ is benzoylamino or alkanoylamino of 2 to 5 carbon atoms, m is the number 1 or 2 and M is hydrogen or an alkali metal.

4. The process as claimed in claim 1, wherein K is a radical of the formula

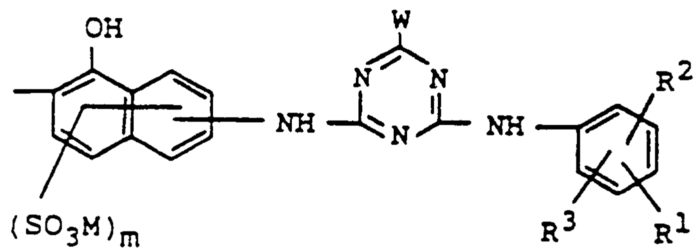

(4v)

in which m is the number 1 or 2, W is sulfo, alkylsulfonyl of 1 to 4 carbon atoms, phenylsulfonyl, bromine, fluorine or chlorine, $R^1$ is the group $-SO_2-Y$ having one of the meanings mentioned in claim 1, $R^2$ is hydrogen, alkyl of 1 to 4 carbon atoms, alkoxy of 1 to 4 carbon atoms, chlorine, bromine, carboxyl, sulfo or nitro, $R^3$ is hydrogen and M is hydrogen or an alkali metal.

5. The process as claimed in claim 1, wherein K is a radical of the formula

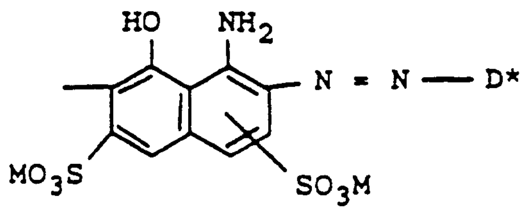

in which D* is phenyl, which can be substituted by 1, 2 or 3 substituents from the group comprising alkyl of 1 to 4 carbon atoms, alkoxy of 1 to 4 carbon atoms, chlorine, bromine, hydroxyl, carboxyl, sulfo, carbamoyl, sulfamoyl and alkanoylamino of 2 to 5 carbon atoms and/or by a group of the formula $-SO_2-Y$ where Y has one of the meanings mentioned in claim 1, or is naphthyl which is substituted by 1, 2 or 3 sulfo groups or by 1 or 2 sulfo groups and 1 or 2 groups of the formula $-SO_2-Y$ where Y has one of the meanings mentioned in claim 1, or only by one of these groups $-SO_2-Y$, and M is a hydrogen atom or an alkali metal.

6. The process as claimed in claim 4, wherein W is fluorine or chlorine.

7. The process as claimed in claim 5, wherein D* is phenyl which is substituted in the para position relative to the azo group by a group of the formula $-SO_2-Y$ where Y has the meaning mentioned in claim 1.

8. The process as claimed in at least one of claims 1 to 7, wherein R is hydrogen.

9. The process as claimed in at least one of claims 1 to 8, wherein Y is vinyl or $\beta$-sulfatoethyl.

10. The process as claimed in at least one of claims 1 to 9, wherein Y is $\beta$-sulfatoethyl.

11. The use of a compound of the formula (1) of claim 1 for the dyeing (including printing) of hydroxyl- and/or carboxamido-containing material, in particular fiber material.

12. A process for the dyeing (including printing) of hydroxyl- and/or carboxamido-containing material, in particular fiber material, in which a dye is applied to the material or incorporated therein and fixed by means of heat and/or by means of an alkaline agent, wherein the dye used is a compound of the formula (1) of claim 1.

**13.** A process for the preparation of a compound of the formula (8) or (5)

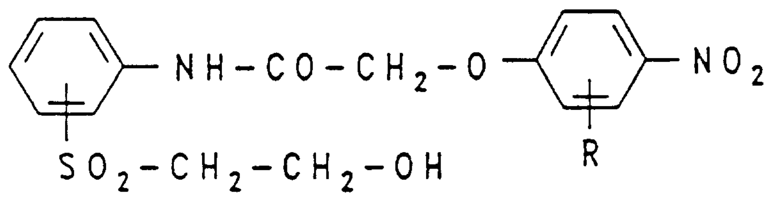

(8)

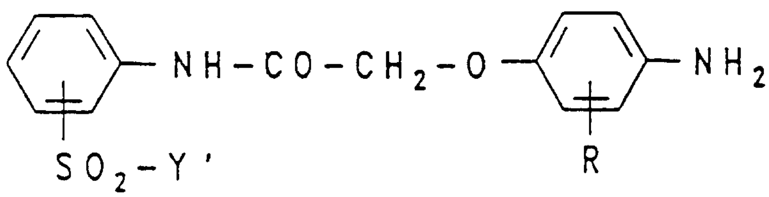

(5)

in which
R is hydrogen, nitro, alkyl of 1 to 4 carbon atoms, alkoxy of 1 to 4 carbon atoms, carboxyl, hydroxyl or halogen, and
Y' is β-hydroxyethyl or vinyl or ethyl containing a substituent in the β-position which can be eliminated by an alkali with the formation of the vinyl group which comprises reacting a compound of the formula (6)

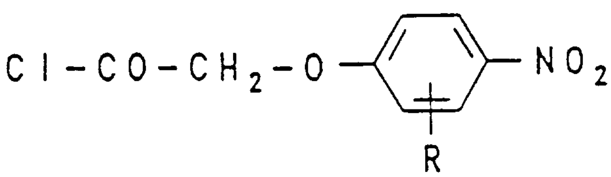

(6)

in which R has the meaning mentioned above with an amino compound of the general formula

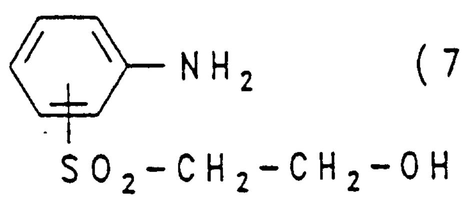

(7)

and reducing the nitro group in the compound of the formula (8) thus obtained to give the compound of the formula (5A)

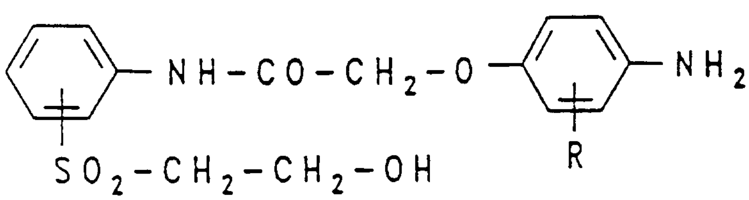

(5A)

where R has the abovementioned meaning and, if desired, converting the β-hydroxyethylsulfonyl group in the compound of the formula (5A) into a group of the formula Y-$SO_2$- where Y is vinyl or ethyl containing a substituent in the β-position which can be eliminated by an alkali with the formation of the

vinyl group.

**14.** The process as claimed in claim 13, wherein Y' is β-hydroxyethyl, β-sulfatoethyl or vinyl.

**15.** The process as claimed in claim 13 or 14, wherein R is hydrogen.

**Revendications**

**Revendications pour les Etats contractants suivants : BE, CH, DE, FR, GB, IT, LI**

**1.** Composé azoïque hydrosoluble répondant à la formule générale (1) :

$$D - N = N - K \quad (1)$$

dans laquelle :

D représente un groupe de formule générale (2)

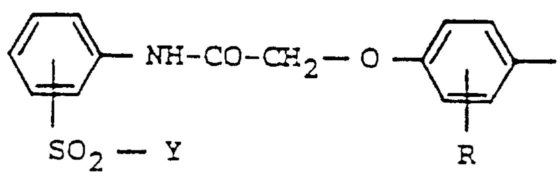

(2)

dans laquelle

Y représente le vinyle ou un groupe de formule générale (3) :

$$-CH_2-CH_2-X \quad (3)$$

dans laquelle

X représente un substituant, éliminable en milieu alcalin en formant le groupe vinyle, et

R représente l'hydrogène, les nitro, alkyle comportant de 1 à 4 atomes de carbone, alcoxy comportant de 1 à 4 atomes de carbone, carboxy, hydroxy ou halogène ;

K est un radical d'un composant de copulation hydrosoluble simplement copulable, qui peut contenir encore un groupe azoïque, ou le radical d'un composant de copulation hydrosoluble, doublement copulable, chaque fois de la série des aminobenzènes, des phénols, plus particulièrement de leurs acides sulfoniques et carboxyliques, des naphtols, plus particulièrement de leurs acides sulfoniques et carboxyliques, des aminonaphtols, plus particulièrement de leurs acides sulfoniques, des acylaminonaphtols, plus particulièrement de leurs acides sulfoniques avec le radical acyle d'un acide alcane- ou alcène-carboxylique, chaque fois comportant de 1 à 4 ou de 2 à 4 atomes de carbone dans le radical alkyle ou alcényle, ou d'un acide carboxylique aromatique ou d'un acide sulfonique aromatique ou un d'acide carbaminique N-substitué ou de la série des acides dihydroxynaphtalène-sulfoniques, des acides phénylazo- et naphtylazo-aminonaphtolsulfoniques, des 5-pyrazolones et 5-aminopyrazoles, des acétoacétyl-arylides, des 2-hydroxy-6-pyridones et des hydroxyquinoléines, K pouvant aussi contenir, outre les substituants usuels dans les colorants, un ou plusieurs groupes réactifs vis-à-vis des fibres.

**2.** Composé selon la revendication 1, caractérisé en ce que K représente un radical de formule générale :

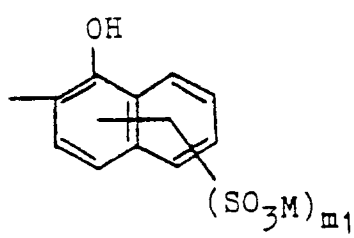

dans laquelle $m_1$ représente le nombre 1, 2 ou 3 et M représente un atome d'hydrogène ou un métal alcalin.

**3.** Composé selon la revendication 1, caractérisé en ce que K représente un radical de formule générale :

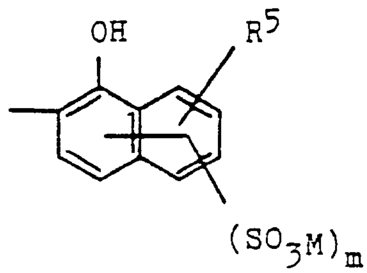

dans laquelle $R^5$ représente le benzoylamino ou un alcanoylamino comportant de 2 à 5 atomes de carbone, m est le nombre 1 ou 2 et M représente l'hydrogène ou un métal alcalin.

**4.** Composé selon la revendication 1, caractérisé en ce que K représente un radical de formule générale :

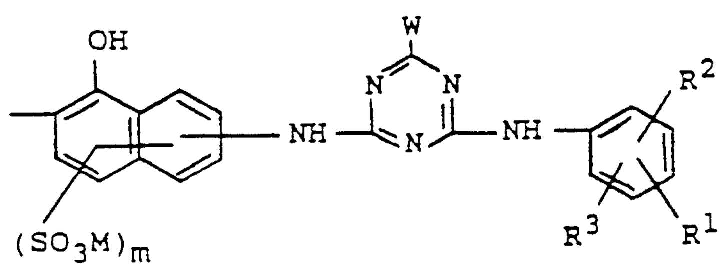

(4v)

dans laquelle m est le nombre 1 ou 2, W représente les sulfo, alkylsulfonyle comportant de 1 à 4 atomes de carbone, phénylsulfonyle, brome, fluor ou chlore, $R^1$ représente le groupe $-SO_2-Y$ avec l'une des significations citées dans la revendication 1, $R^2$ représente l'hydrogène, les alkyle comportant de 1 à 4 atomes de carbone, alcoxy comportant de 1 à 4 atomes de carbone, chlore, brome, carboxy, sulfo ou nitro, $R^3$ représente l'hydrogène et M représente l'hydrogène ou un métal alcalin.

**5.** Composé selon la revendication 1, caractérisé en ce que K représente un radical de formule générale :

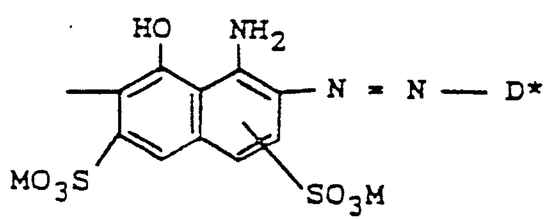

dans laquelle D* est le phényle, qui peut être substitué par 1, 2 ou 3 substituants pris dans le groupe comprenant les alkyle comportant de 1 à 4 atomes de carbone, alcoxy comportant de 1 à 4 atomes de carbone, chlore, brome, hydroxy, carboxy, sulfo, carbamoyle, sulfamoyle et alcanoylamino comportant de 2 à 5 atomes de carbone et/ou par un groupe de formule générale $-SO_2-Y$, Y ayant une signification citée dans la revendication 1, ou représente le naphtyle, qui est substitué par 1, 2 ou 3 groupe sulfo ou par 1 ou 2 groupes sulfo et 1 ou 2 groupes de formule générale $-SO_2-Y$, Y ayant l'une des significations citées dans la revendication 1, ou seulement par un tel groupe $-SO_2-Y$, et M représente un atome d'hydrogène ou un métal alcalin.

**6.** Composé selon la revendication 4, caractérisé en ce que W est le fluor ou le chlore.

7. Composé selon la revendication 5, caractérisé en ce que D* est le phényle, qui est substitué en position para par rapport au groupe azo par un groupe de formule générale $-SO_2-Y$, Y ayant la signification donnée dans la revendication 1.

8. Composé selon au moins l'une des revendications 1 à 7, caractérisé en ce que R est l'hydrogène.

9. Composé selon au moins l'une des revendications 1 à 8, caractérisé en ce que Y est le vinyle ou β-sulfatoéthyle.

10. Composé selon au moins l'une des revendications 1 à 9, caractérisé en ce que Y est le β-sulfatoéthyle.

11. Procédé pour la préparation d'un composé de formule générale (1) citée et définie dans la revendication 1, caractérisé en ce qu'on copule un composé de diazonium d'un composé amino de formule générale (5) :

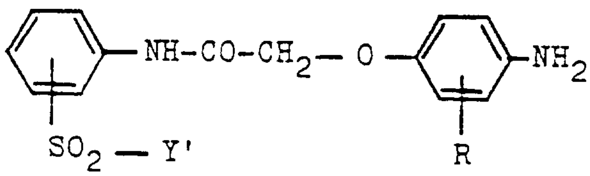

(5)

dans laquelle Y' a l'une des significations de Y ou est le groupe β-hydroxyéthyle et R a la signification citée dans la revendication 1, avec un composant de copulation de formule générale H-K avec K ayant la signification citée dans la revendication 1, et éventuellement, on fait réagir le composé azoïque formé avec un autre composé diazoïque ou un composant de copulation, et si Y' est le groupe β-hydroxyéthyle, celui-ci est transformé dans le composé azoïque formé en un groupe Y ayant la signification citée dans la revendication 1.

12. Utilisation d'un composé répondant à la formule générale (1) de la revendication 1, pour la teinture (y compris l'impression) de matières contenant des groupes hydroxy et/ou carboxamido, plus particulièrement de matières fibreuses.

13. Procédé pour la teinture (y compris l'impression) de matières contenant des groupes hydroxy et/ou carboxamido, plus particulièrement de matières fibreuses, selon lequel on fait absorber le colorant par la matière ou on l'incorpore dans celle-ci, et on le fixe au moyen de la chaleur et/ou à l'aide d'un agent à action alcaline, caractérisé en ce qu'on utilise en tant que colorant un composé répondant à la formule générale (1) de la revendication 1.

14. Composé répondant à la formule générale (8) :

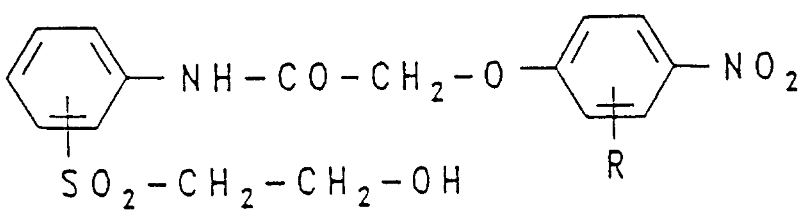

(8)

dans laquelle R représente l'hydrogène, les nitro, alkyle comportant de 1 à 4 atomes de carbone, alcoxy comportant de 1 à 4 atomes de carbone, carboxy, hydroxy ou halogène.

**15.** Composé répondant à la formule générale :

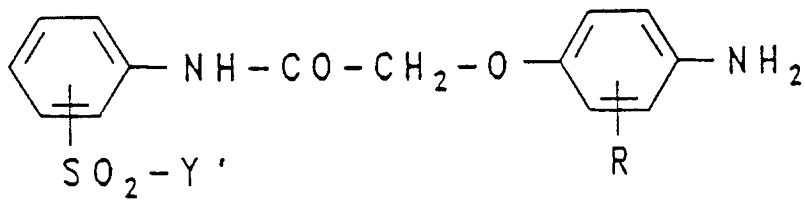

(5)

dans laquelle

R représente les hydrogène, nitro, alkyle comportant de 1 à 4 atomes de carbone, alcoxy comportant de 1 à 4 atomes de carbone, carboxy, hydroxy ou halogène, et

Y' représente le $\beta$-hydroxyéthyle ou le vinyle ou l'éthyle, qui contient en position $\beta$ un substituant éliminable en milieu alcalin en formant le groupe vinyle.

**16.** Composé selon la revendication 15, caractérisé en ce que Y' est le $\beta$-hydroxyéthyle, $\beta$-sulfatoéthyle ou vinyle.

**17.** Composé selon les revendications 15 ou 16, caractérisé en ce que R est l'hydrogène.

**18.** Procédé pour la préparation d'un composé des revendications 14 ou 15, caractérisé en ce qu'on fait réagir un composé de formule générale (6) :

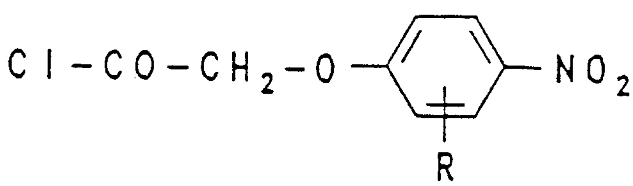

(6)

dans laquelle R a la signification donnée dans la revendication 14, avec un composé amino de formule générale (7) :

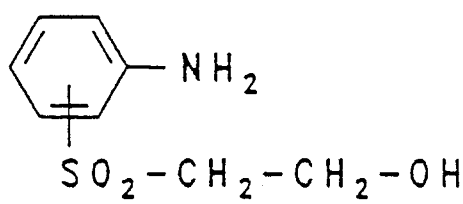

(7)

et qu'on réduit le groupe nitro dans le composé ainsi obtenu de formule générale (8) pour composé de formule générale (5A) :

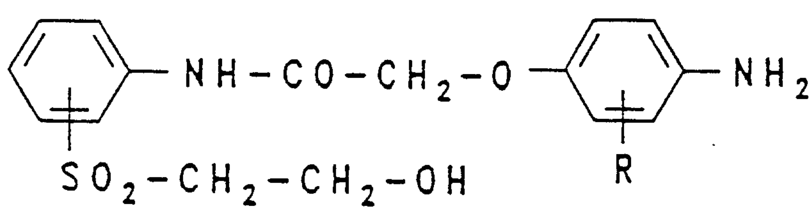

avec R ayant la signification donnée ci-dessus et, éventuellement, dans le composé de formule (5A), on transforme le groupe $\beta$-hydroxyéthyle-sulfonyle en un groupe de formule Y-$SO_2$-, Y ayant une signification citée dans la revendication 1.

**19.** Utilisation d'un composé des revendications 14 ou 15 pour la synthèse de colorants, plus particulièrement de colorants azoïques.

**Revendications pour l'Etat contractant suivant : ES**

**1.** Procédé pour la préparation d'un composé azoïque hydrosoluble répondant à la formule générale (1) :

D — N = N — K (1)

dans laquelle :

D représente un groupe de formule générale (2)

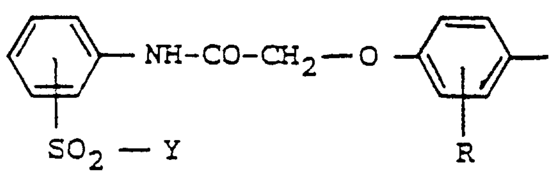

(2)

dans laquelle

Y représente le vinyle ou un groupe de formule générale (3) :

$-CH_2-CH_2-X$ (3)

dans laquelle

X représente un substituant, éliminable en milieu alcalin en formant le groupe vinyle, et

R représente l'hydrogène, les nitro, alkyle comportant de 1 à 4 atomes de carbone, alcoxy comportant de 1 à 4 atomes de carbone, carboxy, hydroxy ou halogène ;

K est un radical d'un composant de copulation hydrosoluble copulable simplement, qui peut contenir encore un groupe azoïque, ou le radical d'un composant de copulation hydrosoluble, doublement copulable, chaque fois de la série des aminobenzènes, des phénols, plus particulièrement de leurs acides sulfoniques et carboxyliques, des naphtols, plus particulièrement de leurs acides sulfoniques et carboxyliques, des aminonaphtols, plus particulièrement de leurs acides sulfoniques, des acylaminonaphtols, plus particulièrement de leurs acides sulfoniques avec le radical acyle d'un acide alcane- ou alcène-carboxylique, chaque fois comportant de 1 à 4 ou de 2 à 4 atomes de carbone dans le radical alkyle ou alcényle, ou d'un acide carboxylique aromatique ou d'un acide sulfonique aromatique ou d'un acide carbaminique N-substitué ou de la série des acides dihydroxynaphtalène-sulfoniques, des acides phénylazo- et naphtylazo-aminonaphtol-sulfoniques, des 5-pyrazolones et 5-aminopyrazoles, des acétoacétyl-arylides, des 2-hydroxy-6-pyridones et des hydroxyquinoléines, K pouvant aussi contenir, outre les substituants usuels dans les colorants, un ou plusieurs groupes réactifs vis-à-vis des fibres,

caractérisé en ce qu'on copule un composé de diazonium d'un composé amino de formule générale (5)

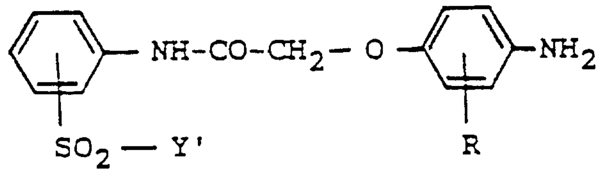

(5)

dans laquelle Y' a l'une des significations de Y ou représente le groupe $\beta$-hydroxyéthyle et R a la signification donnée ci-dessus, avec un composant de copulation de formule générale H-K avec K ayant la signification donnée ci-dessus, et éventuellement on fait réagir le composé azoïque formé avec un autre composé diazoïque ou composant de copulation, et si Y' est le groupe $\beta$-hydroxyéthyle, celui-ci est transformé dans le composé azoïque formé en un groupe Y ayant la signification donnée ci-dessus.

**2.** Procédé selon la revendication 1, caractérisé en ce que K représente un radical de formule générale

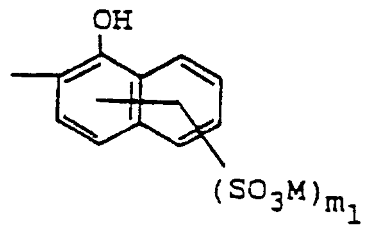

dans laquelle $m_1$ représente le nombre 1, 2 ou 3 et M représente un atome d'hydrogène ou un métal alcalin.

**3.** Procédé selon la revendication 1, caractérisé en ce que K représente un radical de formule générale

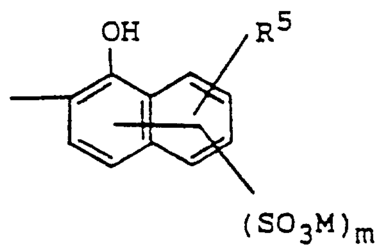

dans laquelle $R^5$ représente le benzoylamino ou un alcanoylamino comportant de 2 à 5 atomes de carbone, m est le nombre 1 ou 2 et M représente l'hydrogène ou un métal alcalin.

**4.** Procédé selon la revendication 1, caractérisé en ce que K représente un radical de formule générale

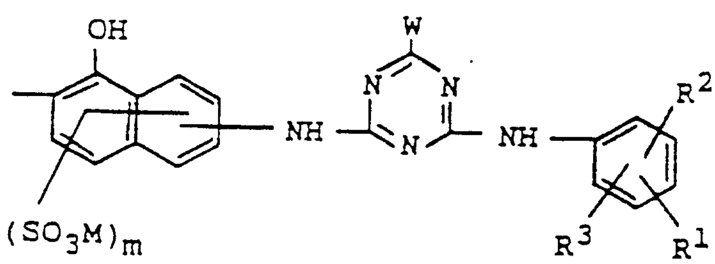

(4v)

dans laquelle m est le nombre 1 ou 2, W représente les sulfo, alkylsulfonyle comportant de 1 à 4 atomes de carbone, phénylsulfonyle, brome, fluor ou chlore, $R^1$ représente le groupe $-SO_2-Y$ ayant l'une des significations citées dans la revendication 1, $R^2$ représente l'hydrogène, les alkyle comportant de 1 à 4 atomes de carbone, alcoxy comportant de 1 à 4 atomes de carbone, chlore, brome, carboxy, sulfo ou nitro, $R^3$ représente l'hydrogène et M représente l'hydrogène ou un métal alcalin.

**5.** Procédé selon la revendication 1, caractérisé en ce que K représente un radical de formule générale :

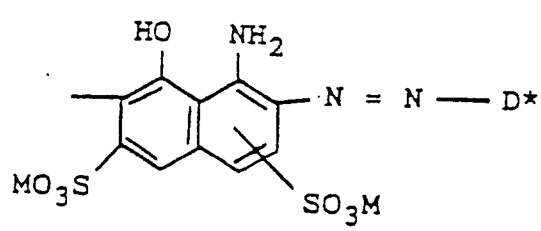

dans laquelle D* est le phényle, qui peut être substitué par 1, 2 ou 3 substituants pris dans le groupe

comprenant les alkyle comportant de 1 à 4 atomes de carbone, alcoxy comportant de 1 à 4 atomes de carbone, chlore, brome, hydroxy, carboxy, sulfo, carbamoyle, sulfamoyle et alcanoylamino comportant de 2 à 5 atomes de carbone et/ou par un groupe de formule générale $-SO_2-Y$, Y ayant une signification citée dans la revendication 1, ou représente le naphtyle, qui est substitué par 1, 2 ou 3 groupe sulfo ou par 1 ou 2 groupes sulfo et 1 ou 2 groupes de formule générale $-SO_2-Y$, Y ayant une signification citée dans la revendication 1, ou est substitué seulement par un tel groupe $-SO_2-Y$, et M représente un atome d'hydrogène ou un métal alcalin.

**6.** Procédé selon la revendication 4, caractérisé en ce que W est le fluor ou le chlore.

**7.** Procédé selon la revendication 5, caractérisé en ce que D* est le phényle, qui est substitué en position para par rapport au groupe azo par un groupe de formule générale $-SO_2-Y$, Y ayant la signification donnée dans la revendication 1.

**8.** Procédé selon au moins l'une des revendications 1 à 7, caractérisé en ce que R est l'hydrogène.

**9.** Procédé selon au moins l'une des revendications 1 à 8, caractérisé en ce que Y est le vinyle ou le β-sulfatoéthyle.

**10.** Procédé selon au moins l'une des revendications 1 à 9, caractérisé en ce que Y est le β-sulfatoéthyle.

**11.** Utilisation d'un composé répondant à la formule générale (1) de la revendication 1 pour la teinture (ou l'impression) de matières contenant des groupes hydroxy et/ou carboxamido, plus particulièrement de matières fibreuses.

**12.** Procédé pour la teinture (y compris l'impression) des matières contenant des groupes hydroxy et/ou carboxamido, plus particulièrement de matières fibreuses, selon lequel on fait absorber le colorant sur la matière ou on l'incorpore dans celle-ci et on le fixe au moyen de la chaleur et/ou à l'aide d'un agent à action alcaline, caractérisé en ce qu'on utilise en tant que colorant un composé répondant à la formule générale (1) de la revendication 1.

**13.** Procédé pour la préparation d'un composé répondant aux formules générales (8) ou (5) :

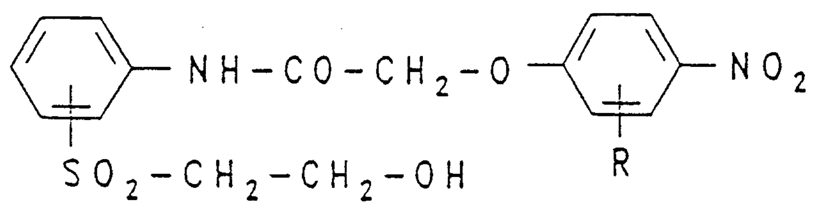
(8)

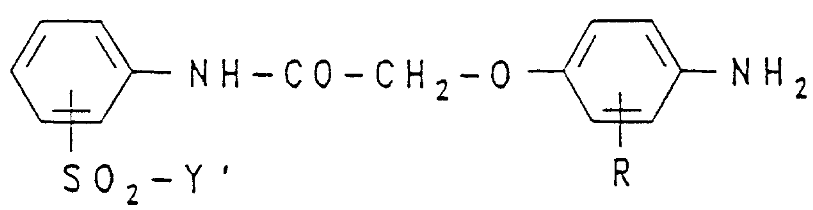
(5)

dans lesquelles

R représente l'hydrogène, les nitro, alkyle comportant de 1 à 4 atomes de carbone, alcoxy comportant de 1 à 4 atomes de carbone, carboxy, hydroxy ou halogène, et

Y' représente le β-hydroxyéthyle ou le vinyle ou est l'éthyle, qui contient en position β un substituant éliminable en milieu alcalin en formant le groupe vinyle,

caractérisé en ce qu'on fait réagir un composé de formule générale (6) :

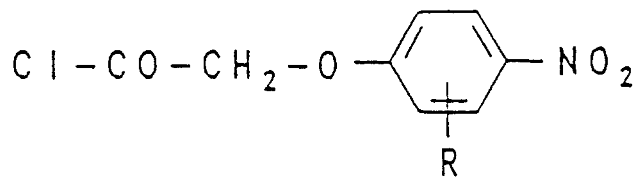

(6)

dans laquelle R a la signification donnée ci-dessus avec un composé amino de formule générale (7) :

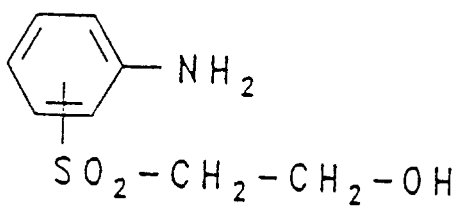

(7)

et on réduit le groupe nitro dans le composé ainsi obtenu de formule générale (8) pour obtenir le composé de formule générale (5A) :

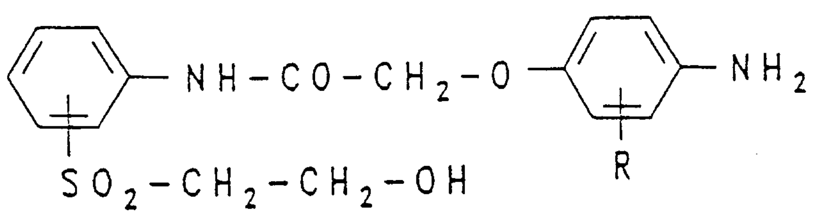

(5A)

avec R ayant la signification donnée ci-dessus et éventuellement on transforme le groupe $\beta$-hydroxyéthylsulfonyle dans le composé de formule (5A) en un groupe de formule Y-$SO_2$-, dans laquelle Y est le vinyle ou l'éthyle, qui contient en position $\beta$ un substituant éliminable en milieu alcalin en formant le groupe vinyle.

**14.** Procédé selon la revendication 13, caractérisé en ce que Y' représente les groupes $\beta$-hydroxyéthyle, $\beta$-sulfatoéthyle ou vinyle.

**15.** Procédé selon la revendication 13 ou 14, caractérisé en ce que R est l'hydrogène.